(19) Europäisches Patentamt

European Patent Office

Office européen des brevets

(11) **EP 4 345 840 A1**

(12) **EUROPEAN PATENT APPLICATION**
published in accordance with Art. 153(4) EPC

(43) Date of publication:
**03.04.2024 Bulletin 2024/14**

(21) Application number: **22832214.5**

(22) Date of filing: **01.07.2022**

(51) International Patent Classification (IPC):
*G16H 40/67* (2018.01)    *G16H 50/70* (2018.01)
*G16H 80/00* (2018.01)    *G16H 10/60* (2018.01)
*G06F 9/54* (2006.01)

(52) Cooperative Patent Classification (CPC):
**G16H 50/70; G16H 40/20**

(86) International application number:
**PCT/CN2022/103328**

(87) International publication number:
**WO 2023/274402 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.07.2021 CN 202110745539**

(71) Applicant: **BOE Technology Group Co., Ltd.
Chaoyang District,
Beijing 100015 (CN)**

(72) Inventors:
• **ZHANG, Xiying
Beijing 100176 (CN)**
• **TIAN, Fuchen
Beijing 100176 (CN)**
• **WANG, Tongbo
Beijing 100176 (CN)**

• **CUI, Can
Beijing 100176 (CN)**
• **WANG, Hongliang
Beijing 100176 (CN)**
• **CHU, Xiao
Beijing 100176 (CN)**
• **ZHANG, Honglei
Beijing 100176 (CN)**
• **WU, Xinyin
Beijing 100176 (CN)**
• **LIU, Xinxin
Beijing 100176 (CN)**
• **TONG, Xiaotong
Beijing 100176 (CN)**
• **ZHAO, Lei
Beijing 100176 (CN)**
• **HU, Yanyang
Beijing 100176 (CN)**

(74) Representative: **Potter Clarkson
Chapel Quarter
Mount Street
Nottingham NG1 6HQ (GB)**

(54) **EMERGENCY TREATMENT SYSTEM, EMERGENCY TREATMENT METHOD, AND ELECTRONIC DEVICE**

(57)    Provided are an emergency treatment system, an emergency treatment method, and an electronic device. The emergency treatment system comprises: an information collection terminal, which is deployed in a rescue vehicle and is used for collecting medical data of a subject to be treated and uploading the medical data to a data lake cluster; the data lake cluster, which is used for performing data access, data processing, data storage and data distribution on the medical data of said object; and a medical business system, which is used for acquiring the medical data of said object from the data lake cluster.

FIG.2

Description

CROSS-REFERENCE

The present application claims the priority to the Chinese Patent Application NO. 202110745539.X, entitled "EMERGENCY TREATMENT SYSTEM, EMERGENCY TREATMENT METHOD, AND ELECTRONIC DEVICE", filed on July 1, 2021, the entire contents of which are hereby incorporated by reference.

TECHNICAL FIELD

The present disclosure relates to the field of smart medical technologies, and in particular to an emergency treatment system, an emergency treatment method and an electronic device.

BACKGROUND

Emergency treatments refer to seeking emergency treatment help by making an emergency call or through other means when a patient suddenly becomes ill outside hospitals. It should be noted that the information disclosed in the Background section above is only for enhancing the understanding of the background of the present disclosure, and thus may include information that does not constitute prior art known to those of ordinary skill in the art.

SUMMARY

The present disclosure provides an emergency treatment system, an emergency treatment method and an electronic device.

According to an aspect of the present disclosure, there is provided an emergency treatment system, including:

an information collection terminal, deployed in an ambulance vehicle, and configured to collect medical data of a subject to be treated and upload it to a data lake cluster;
the data lake cluster, configured to perform data access, data processing, data storage and data distribution for the medical data of the subject to be treated; and
a medical business system, configured to obtain the medical data of the subject to be treated from the data lake cluster.

In an embodiment of the present disclosure, the data lake cluster includes:

a data access module, configured to determine a corresponding target access manner according to a type of the information collection terminal and access the medical data of the subject to be treated in the target access manner;
a data processing module, configured to analyze indicator information of the medical data of the subject to be treated, and establish a mapping relationship between each piece of indicator information and a standard indicator; and
a data distribution module, configured to distribute the medical data of the subject to be treated processed by the data processing module to the medical business system.

In an embodiment of the present disclosure, the analyzing in the data processing module the indicator information of the medical data of the subject to be treated includes:

analyzing, based on a data analysis model, the indicator information of the medical data of the subject to be treated uploaded by the information collection terminal;
wherein the data analysis model is obtained by modeling based on an analytical protocol corresponding to the information collection terminal.

In an embodiment of the present disclosure, the indicator information of the medical data of the subject to be treated includes n fields, wherein an i-th field represents a $(i-1)*m+1$-th bit to an $i*m$-th bit of a binary value of an indicator of the medical data of the subject to be treated, m is a positive integer, representing a number of digits represented by each field in corresponding binary data; and the indicator information of the medical data of the subject to be treated is analyzed by the data analysis model through the following formula:

$$y = \sum_{i=1}^{n} f_2 \left[ f_1(x), i \right] * (2^m)^{i-1}$$

where the function $f_1(x)$ is configured to convert an object into a binary value, and $f_2(x)$ is configured to convert the object into a decimal value.

In an embodiment of the present disclosure, the standard indicator at least includes a standard code identity, and the establishing the mapping relationship between each piece of indicator information and the standard indicator in the data processing module includes:

after analyzing the indicator information of the medical data of the subject to be treated uploaded by the information collection terminal, obtaining a non-standard code identity therein;
based on the mapping relationship between the non-standard code identity and the standard code identity, performing key-value storage for the standard code identity and the indicator value in the indicator information of the medical data of the subject to be treated; and
associating the standard code identity corresponding to the medical data of the subject to be treated with a device identity of the information collection terminal.

In an embodiment of the present disclosure, wherein:

a first message platform is configured between the data access module and the data processing module, and the first message platform is used by the data access module to transmit the accessed information to the data processing module in a message service manner; and
a second message platform is configured between the data distribution module and the medical business system, and the second message platform is used by the data distribution module to transmit the processed medical data of the subject to be treated to the medical business system in the message service manner.

In an embodiment of the present disclosure, the data distribution module is further configured to send the medical data of the subject to be treated to a message queue for the second message platform to pull the medical data of the subject to be treated from the message queue.

In an embodiment of the present disclosure, a data conversion relationship exists between the medical data of the subject to be treated accessed by the data access module and the processed medical data of the subject to be treated distributed by the data distribution module.

In an embodiment of the present disclosure, the input indicator includes a hypopnea index x1, an apnea index x2 and a pressure parameter x3; the output indicator includes a sleep apnea hypopnea index mean y1; and the following data conversion relationship exists between y1 and x1, x2 and x3:
y1=f1(x1,x2,x3); where f1 represents a linear conversion relationship or a nonlinear conversion relationship.

In an embodiment of the present disclosure, the data conversion relationship is:
y1=w1*x1+w2*x2+w3*x3; where, w1, w2 and w3 are relational parameters.

In an embodiment of the present disclosure, a value range of w1 is [1,1.00000011], a value range of w2 is [1,1.00000011], and a value range of w3 is [7.85046229E -18,7.85046229E-16].

In an embodiment of the present disclosure, a value of w1 is 1.00000011, a value of w2 is 1.00000011, and a value of w3 is 7.85046229E-17.

In an embodiment of the present disclosure, the input indicator includes a hypopnea index x1, an apnea index x2 and a pressure parameter x3; the output indicator is an apnea index mean y2; and the following data conversion relationship exists between y2 and x1, x2 and x3:
y2=f2(x1,x2,x3); where f2 represents a linear conversion relationship or a nonlinear conversion relationship.

In an embodiment of the present disclosure, the data conversion relationship is:
y2=w1*x1+w2*x2+w3*x3; where, w1, w2 and w3 are relational parameters.

In an embodiment of the present disclosure, a value range of w1 is [5.55500801E-08, 0.50000006], a value range of w2 is [0.50000006, 1.00000006], and a value range of w3 is [2.22044605E-17, 2.22044605E-15].

In an embodiment of the present disclosure, a value of w1 is 0.50000006, 0.500000056 or 5.55500801E-08, a value of w2 is 0.50000006 or 1.00000006, and a value of w3 is 2.22044605E-16.

In an embodiment of the present disclosure, the input indicator includes a hypopnea index x1, an apnea index x2 and a pressure parameter x3; the output indicator is a hypopnea index mean y3; and the following data conversion relationship exists between y3 and x1, x2 and x3:
y3=f3(x1,x2,x3); where f3 represents a linear conversion relationship or a nonlinear conversion relationship.

In an embodiment of the present disclosure, the data conversion relationship is:

$y3=w1*x1+w2*x2+w3*x3$; where, w1, w2 and w3 are relational parameters.

In an embodiment of the present disclosure, a value range of w1 is [0.500000056, 1.00000006], a value range of w2 is [5.55500802E-08, 0.50000006], and a value range of w3 is [-7.85046229E-16, - 7.85046229E-18].

In an embodiment of the present disclosure, a value of w1 is 0.50000006, 0.500000056 or 1.00000006, a value of w2 is 5.55500802E-08 or 0.50000006, and a value of w3 is -7.85046229E-17.

In an embodiment of the present disclosure, the data conversion relationship between the medical data of the subject to be treated accessed by the data access module and the processed medical data of the subject to be treated distributed by the data distribution module is obtained in the following manner:

> obtaining an original mapping matrix R between sample medical data and sample business data, wherein the original mapping matrix R is an M$\times$N matrix, and an element R(i,j) is used to represent a relationship between an input indicator i in the sample medical data and an output indicator j in the sample business data;
> for a number of dimensions k, obtaining a first matrix P of M$\times$k and a second matrix Q of k$\times$N with P$\times$Q=R as a target, and calculating a target mapping matrix Rk based on the first matrix P and the second matrix Q; and
> determining a data conversion relationship between each input indicator and an output indicator in business data according to the original mapping matrix and at least one target mapping matrix;
> where m and n are integers greater than 1, i, j, and k are positive integers, and i $\in$ [1,m], j E [1,n], and k $\in$ [1,m-1].

In an embodiment of the present disclosure, the data conversion relationship between each input indicator and the output indicator in the business data is determined according to the original mapping matrix and 1st to (n-1)-th target mapping matrices.

In an embodiment of the present disclosure, the data conversion relationship is:

$yj=f(xi, i \in [1,m])$; where, yj is a value of the output indicator j, and a parameter of the function f is determined based on the original mapping matrix and an element value of a column where the output indicator j is located in the at least one target mapping matrix, and xi is a value of the input indicator i.

In an embodiment of the present disclosure, the input indicator includes a hypopnea index x1, an apnea index x2 and a pressure parameter x3; the output indicator is a sleep apnea hypopnea index mean y1; and a conversion relationship corresponding to the sleep apnea hypopnea index mean is:

$y1=f1(x1,x2,x3)$; where, a parameter of function f1 is determined based on the original mapping matrix and an element value of a column where the sleep apnea hypopnea index mean is located in the at least one target mapping matrix.

In an embodiment of the present disclosure, the conversion relationship corresponding to the sleep apnea hypopnea index mean is specifically:

$y1=w1*x1+w2*x2+w3*x3$; wherein w1, w2 and w3 are determined based on the original mapping matrix and the element value of the column where the sleep apnea hypopnea index is located in the at least one target mapping matrix.

In an embodiment of the present disclosure, the input indicator includes a hypopnea index x1, an apnea index x2 and a pressure parameter x3, the output indicator is an apnea index mean y2, and a conversion relationship corresponding to the apnea index mean is:

$y2=f2(x1,x2,x3)$; wherein a parameter of the function f2 is determined based on the original mapping matrix and an element value of a column where the apnea index mean is located in the at least one target mapping matrix.

In an embodiment of the present disclosure, the conversion relationship corresponding to the apnea index mean is specifically:

$y2=w1*x1+w2*x2+w3*x3$; wherein w1, w2 and w3 are determined based on an element value of the column in which the apnea index mean is located in the original mapping matrix and at least one of the target mapping matrices.

In an embodiment of the present disclosure, the input indicator includes a hypopnea index x1, an apnea index x2 and a pressure parameter x3, the output indicator is a hypopnea index meany3, and a conversion relationship corresponding to the hypopnea index mean is:

$y3=f3(x1,x2,x3)$, wherein a parameter of the function f3 is determined based on the original mapping matrix and an element value of a column where the hypopnea index mean is located in the at least one target mapping matrix.

In an embodiment of the present disclosure, the conversion relationship corresponding to the hypopnea index mean is specifically:

$y3=w1*x1+w2*x2+w3*x3$; wherein w1, w2 and w3 are determined based on an element value of a column where the hypopnea index mean is located in the original mapping matrix and at least one of the target mapping matrices.

In an embodiment of the present disclosure, the emergency treatment system further includes a management terminal;

> the management terminal is configured to: record a device start of use time and a device identity of the information collection terminal;

receive medical data distributed from the data lake cluster; and
associate the medical data with user information of the subject to be treated according to the device identity and the device start of use time;
the medical business system is further configured to receive the medical data associated with the user information of the subject to be treated.

In an embodiment of the present disclosure, the management terminal is further configured to store historical medical data of the subject to be treated, and jointly distribute the historical medical data and real-time medical data of the subject to be treated to the medical business system.

In an embodiment of the present disclosure, the management terminal is further configured to store medical data of relatives and friends of the subject to be treated, and jointly distribute the medical data of the relatives and friends of the subject to be treated and the medical data of the subject to be treated to the medical business system.

In an exemplary embodiment of the present disclosure, the management terminal is further configured to receive a query request, and obtain, according to a query condition included in the query request, relevant data from the data lake cluster to generate a query result.

In an embodiment of the present disclosure, the management terminal further includes:
a data visualization module, configured to generate visual content according to data of the data lake cluster to display the visual content through a front-end page.

In an embodiment of the present disclosure, the management terminal further includes:
a device parameter monitoring module, configured to record and analyze behavior information of the information collection terminal to determine whether the information collection terminal is an abnormal terminal.

In an embodiment of the present disclosure, the emergency treatment system further includes:
a dispatch center terminal, configured to receive alarm information and obtain a position of the subject to be treated, and dispatch, according to a preset dispatch algorithm, an ambulance vehicle to the position of the subject to be treated for treatment.

In an embodiment of the present disclosure, the dispatch center terminal is further configured to dispatch, according to the medical data related to the subject to be treated, an internal rescuer of a medical institution to provide remote assistance.

In an embodiment of the present disclosure, the emergency treatment system further includes:
an alarm terminal, available for being in a communication connection with the dispatch center terminal, and configured to send alarm information to the dispatch center terminal.

In an embodiment of the present disclosure, the emergency treatment system further includes:
a medical treatment personnel terminal, communicatively connected with the dispatch center terminal, and configured to receive the dispatch information of the dispatch center terminal, and enter medical record information of the subject to be treated.

In an embodiment of the present disclosure, the emergency treatment system further includes:
a driver terminal, communicatively connected with the dispatch center terminal and configured to guide the driver to drive the ambulance vehicle to the position of the subject to be treated.

In an embodiment of the present disclosure, the emergency treatment system further includes:

an information integration platform in communication with at least one of the management terminal, the dispatch center terminal, the alarm terminal, the medical treatment personnel terminal, the driver terminal and the medical business system;
the information integration platform is configured to retrieve a personal health file and/or a knowledge base from a third-party business system and send them to the at least one of the management terminal, the dispatch center terminal, the alarm terminal, the medical treatment personnel terminal, the driver terminal and the medical business system; and/or,
the information integration platform is configured to obtain information of the subject to be treated from the at least one of the management terminal, the dispatch center terminal, the alarm terminal, the medical treatment personnel terminal, the driver terminal and the medical business system.

In an embodiment of the present disclosure, the information integration platform includes a decision evaluation module; and
the decision evaluation module is configured to evaluate the information of the subject to be treated based on the knowledge base.

In an embodiment of the present disclosure, the information integration platform is configured to:

identify an emergency scene according to the received information of the subject to be treated; and

upon receiving new alarm information, if it is determined that the new alarm information is located in the identified emergency scene, determining whether to configure a new ambulance vehicle according to an ambulance vehicle configured for the identified emergency scene.

In an embodiment of the present disclosure, the rescuer terminal is further configured to record treatment information, and
the information integration platform is configured to draw a treatment information record as a timeline according to a chronological order and send it to the medical business system.

In an embodiment of the present disclosure, the information integration platform is configured to receive hospital resource information sent from the medical business system and send it to the dispatch center terminal.

According to an aspect of the present disclosure, there is provided an emergency treatment method, including:

collecting, by an information collection terminal deployed in an ambulance vehicle, medical data of a subject to be treated and uploading it to a data lake cluster;
performing, by the data lake cluster, data access, data processing, data storage and data distribution for the medical data of the subject to be treated; and
obtaining, by a medical business system, the medical data of the subject to be treated from the data lake cluster.

According to an aspect of the present disclosure, there is provided an electronic device, including:

a processor; and
a memory, configured to store one or more programs, which when executed by the processor, cause the processor to implement the method as provided in some aspects of the present disclosure.

According to an aspect of the present disclosure, there is provided a computer-readable storage medium having a computer program stored thereon, which, when executed by a processor, implements the method as provided in some aspects of the present disclosure.

It should be noted that the above general description and the following detailed description are merely exemplary and explanatory and should not be construed as limiting of the disclosure.

## BRIEF DESCRIPTION OF THE DRAWINGS

The accompanying drawings, which are incorporated in and constitute a part of this specification, illustrate embodiments consistent with the present disclosure and together with the description serve to explain the principles of the present disclosure. Apparently, the drawings in the following description are only some embodiments of the present disclosure, and those skilled in the art can obtain other drawings according to these drawings without creative efforts.

FIG. 1 shows a schematic architectural diagram of an emergency treatment system according to an embodiment of the present disclosure.
FIG. 2 shows a schematic module diagram of a data lake cluster according to an embodiment of the present disclosure.
FIG. 3 shows a schematic architectural diagram of an emergency treatment system according to an embodiment of the present disclosure.
FIG. 4 shows a schematic diagram of a processing flow of a data processing module according to an embodiment of the present disclosure.
FIG. 5 shows a schematic diagram of a processing flow of a data processing module according to an embodiment of the present disclosure..
FIG. 6 shows a schematic diagram of an indicator code identity mapping relationship according to an embodiment of the present disclosure.
FIG. 7 shows a schematic module diagram of a data lake cluster according to an embodiment of the present disclosure.
FIG. 8 shows a schematic diagram of a processing flow of a data query module according to an embodiment of the present disclosure.
FIG. 9 shows a schematic diagram of a processing flow of a data interaction module according to an embodiment of the present disclosure.
FIG. 10 shows a schematic diagram of a processing flow of a model acquisition module according to an embodiment of the present disclosure.
FIG. 11 shows a schematic architectural diagram of an emergency treatment system according to an embodiment of the present disclosure.

FIG. 12 shows a schematic diagram of part of modules of an emergency treatment system according to an embodiment of the present disclosure.

FIG. 13 shows a schematic flowchart of an emergency treatment method according to an embodiment of the present disclosure.

FIG. 14 shows a schematic structural diagram of a computer system for implementing an electronic device according to an embodiment of the present disclosure.

## DETAILED DESCRIPTION

Example embodiments will now be described more fully with reference to the accompanying drawings. However, the embodiments can be implemented in a variety of forms and should not be construed as being limited to examples set forth herein; rather, these embodiments are provided so that the present disclosure will be more complete and comprehensive so as to convey the idea of the example embodiments to those skilled in this art. The described features, structures, or characteristics may be combined in any suitable manner in one or more embodiments.

In addition, the drawings are merely schematic representations of the present disclosure and are not necessarily drawn to scale. The same reference numerals in the drawings denote the same or similar parts, and the repeated description thereof will be omitted. Some of the block diagrams shown in the figures are functional entities and do not necessarily correspond to physically or logically separate entities. These functional entities may be implemented in software, or implemented in one or more hardware modules or integrated circuits, or implemented in different networks and/or processor devices and/or microcontroller devices.

It should be noted that the terms "include", "configured with" and "provided" in the present disclosure are used to indicate a non-exclusive inclusion and mean that there may be additional elements/components/etc. in addition to the listed elements/components/etc.

There is information isolation between hospitals and ambulance vehicles in conventional emergency treatments. In some instances, some emergency equipment equipped on the ambulance vehicles can provide medical data transmission functions, but there is still a lack of unified management capabilities for medical data.

In an emergency treatment system of embodiments of the present disclosure, scattered medical data of a subject to be treated is transmitted to a data lake cluster for integration, thereby enabling storage of structured medical data, semi-structured medical data and unstructured medical data at any scale. In addition, various roles in the emergency treatment system, such as medical staff, data developers, and business analysts, are allowed to access data through analysis tools and frameworks of their choice, so as to collaboratively process and analyze the data in different manners. In turn, the emergency treatment system in embodiments of the present disclosure can support a medical institution to provide a more precise treatment plan for the subject to be treated.

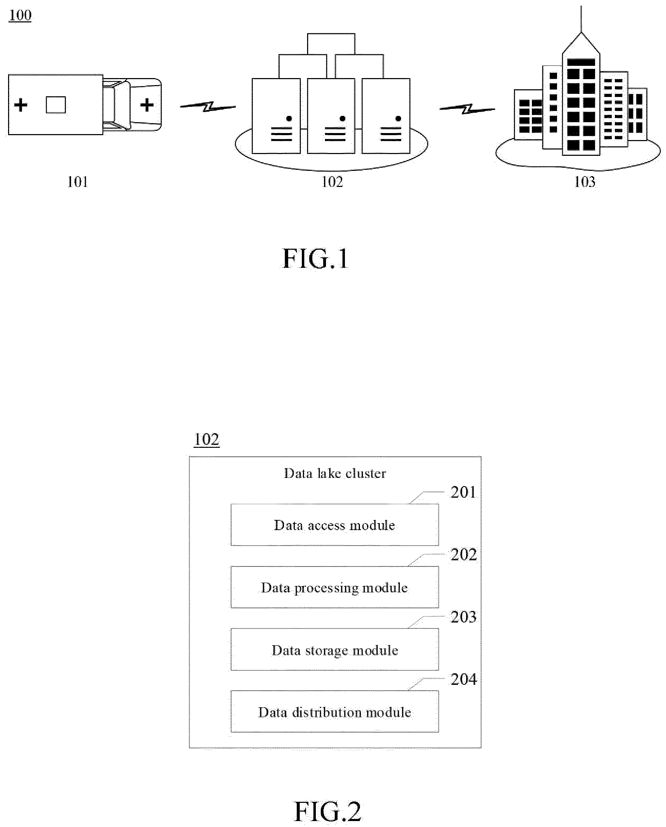

FIG. 1 shows a schematic diagram of an illustrative architecture of an emergency treatment system 100 to which embodiments of the present disclosure can be applied. As shown in FIG. 1, the emergency treatment system 100 may include an information collection terminal deployed in an ambulance vehicle 101, a data lake cluster 102, and a medical business system 103 deployed in a hospital.

In addition to transporting the medical staff and the subject to be treated, the ambulance vehicle 101 is usually used to deploy vehicle-mounted emergency medical instruments. For example, the vehicle-mounted emergency medical instruments may include: a defibrillation monitor, a ventilator, a non-invasive multi-parameter detector (MTX), a blood pressure testing device, a body fat testing device, a blood glucose testing device, a sleep monitoring device, an electric suction device, a negative pressure system, an oxygen supply device, a sterilization device, a lighting device, an exhaust system, a heating system, a power supply line system, an automatic onboard stretcher system, etc. Some vehicle-mounted emergency medical instruments are capable of collecting medical data of the subject to be treated, that is, these vehicle-mounted emergency medical instruments serve as the information collection terminal as described below. The information collection terminal may include, for example, the above-mentioned ventilator, non-invasive multi-parameter detector (MTX), blood pressure testing device, body fat detection device, blood glucose testing device, sleep monitoring device, etc. In addition, some electronic devices of some subjects to be treated, such as a wearable device or other mobile terminals with a medical data collection function, may also be regarded as the information collection terminal described in embodiments of the present disclosure.

The information collection terminal collects the medical data of the subject to be treated and uploads it to the data lake cluster 102. The data lake cluster 102 is used for data access, data processing, data storage and data distribution of the medical data of the subject to be treated. In embodiments of the present disclosure, the data lake cluster 102 may be, for example, a data storage and management service system composed of multiple types of databases, including a relational database such as MariaDB, MySQL, etc., a document database such as MongoDB, CouchDB, etc., a distributed file system such as HDFS, PVFS, PanFS, etc., and a graph database such as Neo4j, Cayley, rapgDB, etc. In embodiments of the present disclosure, the data lake cluster 102 may adopt a distributed computing and storage architecture, integrate various types of computer stand-alone machines, servers and computer clusters or server clusters with data storage and computing functions, and provide various types of functional components including data management and algorithm development. Through the data lake cluster 102, it is possible to store the structured medical data, the semi-structured medical data and the unstructured medical data at any scale without the need for structured processing of the medical data, allow various roles in the emergency treatment system 100, such as the medical staff, the data developers, and the business analysts, to access the data through the analysis tools and frameworks of their choice, so as to collaboratively process and analyze the data in different manners.

In embodiments of the present disclosure, the medical business system 103 can obtain the medical data of the subject to be treated from the data lake cluster 102. For example, the medical business system 103 may be deployed in a medical institution such as the hospital for use by the medical staff.

In the emergency treatment system of embodiments of the present disclosure, the scattered medical data of the subject to be treated is transmitted to the data lake cluster for integration, thereby enabling storage of the structured medical data, the semi-structured medical data and the unstructured medical data at any scale. In addition, various roles in the emergency treatment system, such as the medical staff, the data developers, and the business analysts, are allowed to access the data through the analysis tools and frameworks of their choice, so as to collaboratively process and analyze the data in different manners. In turn, the emergency treatment system in embodiments of the present disclosure can support the medical institution to provide the more precise treatment plan for the subject to be treated.

The emergency treatment system of embodiments of the present disclosure is introduced in more detail below.

Referring to FIG. 2, the data lake cluster 102 includes a data access module 201, a data processing module 202 and a data distribution module 204 in an embodiment of the present disclosure. In some implementations, the data lake cluster 102 may further include a data storage module 203.

The data access module 201 is configured to determine a corresponding target access manner according to a type of the information collection terminal and access the medical data of the subject to be treated in the target access manner.

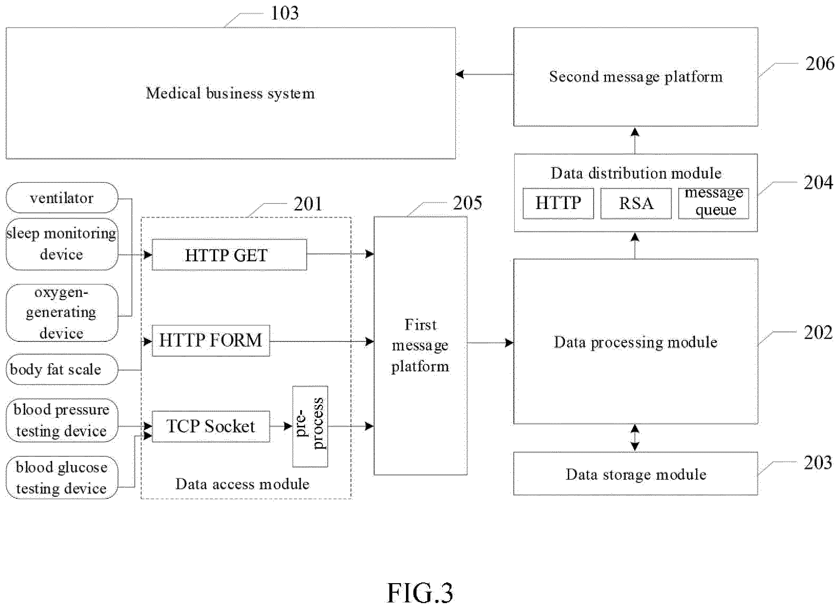

In embodiments of the present disclosure, types of respective information collection terminals and corresponding target access manners may be configured and stored in advance. When a specific information collection terminal accesses, its corresponding target access manner is determined based on preconfigured information. For example, as shown in FIG. 3, if the information collection terminal is a type of device such as the ventilator, the sleep monitoring device, an oxygen-generating device, etc., it may be determined based on the preconfigured information that its corresponding target access manner is a HTTP GET (which requests data from a specified resource based on a hypertext transfer protocol) manner, and then the HTTP GET manner may be employed to access the medical data of the subject to be treated. If the information collection terminal is a type of device such as a body fat scale, etc., it may be determined based on the preconfigured information that its corresponding target access manner is a HTTP FORM (form transfer based on the hypertext transfer protocol) manner, and then the HTTP FORM manner may be employed to access the medical data of the subject to be treated. If the information collection terminal is a type of device such as the blood pressure testing device, the blood glucose testing device, etc., it may be determined based on the preconfigured information that its corresponding target access manner is a TCP Socket (socket transmission based on a transmission control protocol) manner, and then the TCP Socket manner may be employed to access the medical data of the subject to be treated. After the TCP Socket manner is employed to access the medical data of the subject to be treated, the medical data of the subject to be treated may also be pre-processed in a specified manner. Alternatively, in other embodiments of the present disclosure, for the information collection terminal, other access manners such as a Hypertext Transfer Protocol Secure (HTTPS) manner, a User Datagram Protocol (UDP) manner, etc. may also be employed to access the medical data of the subject to be treated, and embodiments of the present disclosure are not limited to this.

The data processing module 202 is configured to analyze indicator information of the medical data of the subject to be treated, and establish a mapping relationship between each piece of indicator information and a standard indicator.

The indicator information is information related to a user's physiological indicator collected by the information collection terminal. In some implementations, the indicator information may include an indicator name and an indicator value.

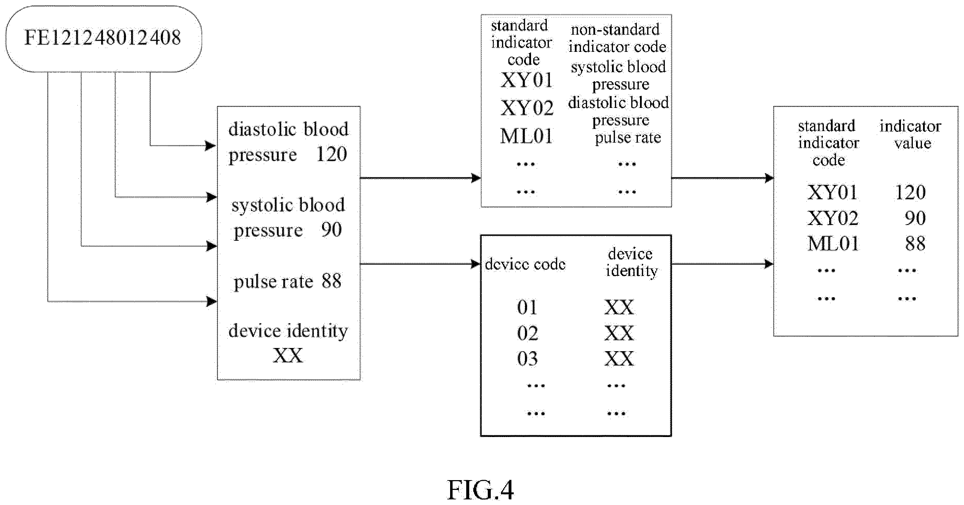

Specifically, since communication protocols, interfaces, data formats, etc. collected by different types of information collection terminals may be different, they need to be analyzed first. In some implementations, analyzing the indicator information includes analyzing the indicator value in the indicator information. For example, as shown in FIG. 4, indicator information collected by a wearable electronic device is hexadecimal data, such as FE121248012408, which needs to be converted into a specific physiological indicator, such as a diastolic blood pressure 120, a systolic blood pressure 90, a pulse rate 88 and so on.

In order to improve an analyzing efficiency of the indicator information of the medical data of the subject to be treated and reduce pre-configuration work for the analyzing manner, reused parts of analyzing manners of some information collection terminals are also extracted for modeling in embodiments of the present disclosure. In some implementations, the indicator information of the medical data of the subject to be treated uploaded by the information collection terminal is analyzed based on a data analysis model. The data analysis model is obtained by performing modeling based on an analytical protocol corresponding to the information collection terminal. In some implementations, analyzing in the data processing module the indicator information of the medical data of the subject to be treated may include: obtaining analytical protocols corresponding to a plurality of information collection terminals, and performing modeling based on an analytical protocol corresponding to each information collection terminal to obtain a data analysis model that is at least partially universal; and analyzing, based on the data analysis model, the indicator information of the medical data of the subject to be treated uploaded by the information collection terminal to which the data analysis model is applicable.

In an embodiment of the present disclosure, the indicator information of the medical data of the subject to be treated includes n fields, and the i-th field represents the (i-1)*m+1-th bit to the i*m-th bit of a binary value of an indicator of the medical data of the subject to be treated, where m is a positive integer and may be determined according to a conversion of number systems relationship. The data analysis model analyzes the indicator information of the medical data of the subject to be treated through the following formula:

$$y = \sum_{i=1}^{n} f_2\left[f_1(x), i\right] * (2^m)^{i-1}$$

where the function $f_1(x)$ is used to convert an object into a binary value, and $f_2(x)$ is used to convert the object into a decimal value.

For example, indicator information sent by a blood pressure testing device is a string 0x00,0x78,0x00,0x50,0x4B. In a data interface protocol provided by a manufacturer of the blood pressure testing device, meanings represented by characters in the string data have been defined. Reference is made to Table 1 below:

Table 1

| Blood pressure value (5byte) | Dat4 | Dat3 | Dat2 | Dat1 | Dat0 |
| --- | --- | --- | --- | --- | --- |
| | SYS[15:8] | SYS[7:0] | DIA[15:8] | DIA[7:0] | PUL [7:0] |

where, SYS[15 : 8] represents upper eight bits of the systolic blood pressure, and SYS[7 : 0] represents lower eight bits of the systolic blood pressure; DIA[15 : 8] represents upper eight bits of the diastolic blood pressure, and DIA[7 : 0] represents lower eight bits of the diastolic blood pressure; and PUL[7 : 0] represents lower eight bits of the pulse rate. Taking the systolic blood pressure as an example, the systolic blood pressure includes two fields (i.e. 0x00, 0x78), where the first field represents the 1st to 8th bits of a binary value of the systolic blood pressure; in this case, m=8, and then a data analysis model corresponding to the systolic blood pressure is:

$$y = \sum_{i=1}^{2} f_2\left[f_1(x), i\right] * 2^{8^{i-1}}$$

Then, the function $f_1(x)$ is used to convert SYS[7:0], that is, 0x78, into the binary value 01111000, and the function $f_2(x)$ is used to convert 01111000 into the decimal value 120. Likewise, it may be obtained that a decimal value corresponding to SYS[15 : 8], that is, 0x00, is 0. As a result, the systolic blood pressure y=0*256^1+120*256^0=120mmHg. Similarly, it may be obtained that the diastolic blood pressure is 80mmHg and a pulse rate is 75 beats/minute. It should be noted that, those skilled in the art can easily understand that in other embodiments of the present disclosure, other data analysis models may also be obtained based on different interface protocols of the information collection terminals, which also fall within the protection scope of the present disclosure.

With continued reference to FIG. 4, after the indicator information of the medical data of the subject to be treated is analyzed, the mapping relationship between each piece of indicator information and the standard indicator may be established.

In some embodiments, the standard indicator at least includes a standard code identity.

After the indicator information of the medical data of the subject to be treated uploaded by the information collection terminal is analyzed, a non-standard code identity therein is obtained.

Based on the mapping relationship between the non-standard code identity and the standard code identity, the standard code identity and the indicator value in the indicator information of the medical data of the subject to be treated are key-value stored.

The standard code identity corresponding to the medical data of the subject to be treated is associated with a device identity of the information collection terminal.

In some embodiments, a process of configuring the standard indicator may further be included.

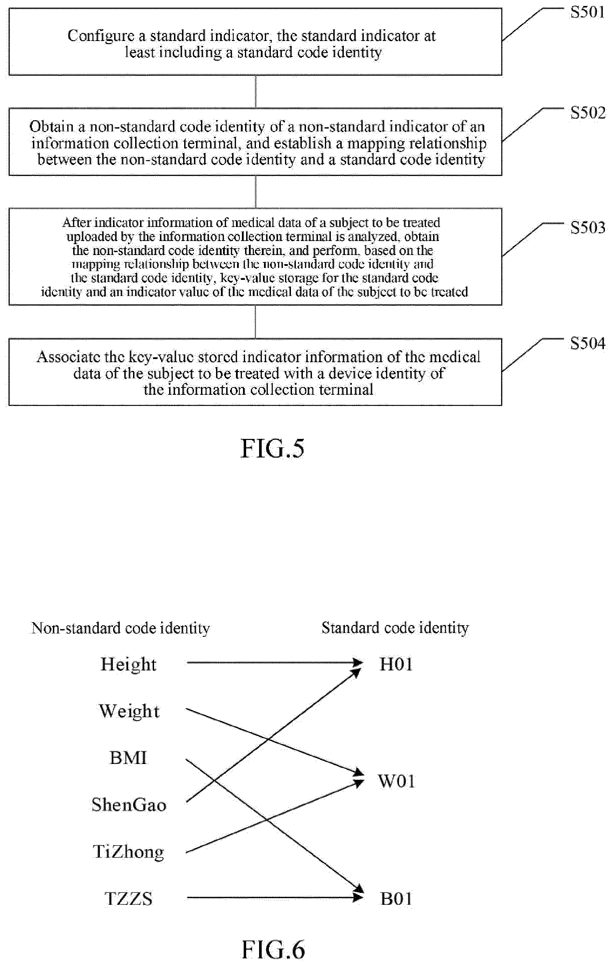

Referring to FIG. 5, establishing the mapping relationship between each piece of indicator information and the standard indicator in the data processing module may include steps S501 to S504.

In the step S501, the standard indicator is configured, and the standard indicator at least includes the standard code identity.

For example, a standard indicator classification may be created, and is associated with a definition of a specific standard indicator in embodiments of the present disclosure. As an example, standard indicators for the blood pressure and the blood glucose are as follows.

Table 2

| Standard indicator type code | Standard indicator type | Standard indicator code | Standard indicator name |
|---|---|---|---|
| CJ00001 | blood pressure | XY01 | systolic blood pressure |
| | | XY02 | diastolic blood pressure |
| CJ00002 | blood glucose | XT01 | fasting blood glucose |
| | | XT02 | pre-meal blood glucose |
| | | XT03 | blood glucose 1 hour after meal |
| | | XT04 | blood glucose 2 hours after meal |

In the step S502, the non-standard code identity of the indicator information of the medical data of the subject to be treated uploaded by the information collection terminal is obtained, and the mapping relationship between the non-standard code identity and the standard code identity is established.

For example, device information of the information collection terminal may be obtained in an embodiment, which may include, for example, basic information (such as a product name, a product type, a product model, a manufacturer name, a product picture), configuration information (such as a communication protocol, a data address, a data format, an interface protocol, an indicator rule, a time rule), etc. Then, the type of the information collection terminal may be determined based on the information such as the product type and the product model; the data uploaded by the information collection terminal may be obtained based on the information such as the communication protocol and the data address; the non-standard code identity may be analyzed from the uploaded data based on the information such as the data format, and the non-standard code identity is, for example, the indicator name in the indicator information; and then the mapping relationship between the non-standard code identity and the standard code identity may be established. For example, as shown in FIG. 6, the non-standard code identities Height and ShenGao both correspond to the standard indicator identity H01; the non-standard code identities Weight and TiZhong both correspond to the standard indicator identity WO1; and the non-standard code identities BMI and TZZS both correspond to the standard indicator identity B01, etc. In addition, in other embodiments of the present disclosure, a user input may be monitored through a front-end control, and then the mapping relationship between the non-standard code identity and the standard code identity is adjusted according to the user input.

In the step S503, after the indicator information of the medical data of the subject to be treated uploaded by the information collection terminal is analyzed, the non-standard code identity therein is obtained, and the standard code identity and the indicator value of the medical data of the subject to be treated are key-value stored based on the mapping relationship between the non-standard code identity and the standard code identity. For example, after the indicator information is analyzed, it is obtained that the systolic blood pressure SYS=120mmHg and the diastolic blood pressure DIA=80mmHg. A standard code identity corresponding to a non-standard code identity SYS is XY01, and a standard code identity corresponding to a non-standard code identity DIA is XY02, then the indicator information is stored as [XY01:120] and [XY02:80] in a key-value form.

In the step S504, the key-value stored indicator information of the medical data of the subject to be treated is associated with the device identity of the information collection terminal. In embodiments of the present disclosure, when the data uploaded by the information collection terminal is received, the device information of the information collection terminal is first matched with device information pre-collected in the above step S502, thereby ensuring that the indicator information of the medical data of the subject to be treated is in a one-to-one correspondence with each information collection terminal.

In some implementations, the standard indicator configuration process and the mapping relationship pre-building process involved in the above steps S501 and S502 may be omitted, and an execution order of the steps S503 and S504 is not limited.

In addition, with continued reference to FIG. 3, in an embodiment of the present disclosure, a first message platform 205 is further configured between the data access module 201 and the data processing module 202, and the first message platform 205 is used by the data access module 201 to transmit the accessed information to the data processing module 202 in a message service manner. For example, the data accessed by the data access module 201 may first be written into a message queue such as RabbitMQ, ActiveMQ, ZeroMQ, Kafka, MetaMQ, RocketMQ, etc., and the data processing module 202 may pull data from the message queue in a first-in-first-out manner for processing. By configuring the first message platform between the data access module and the data processing module, decoupling between the data access module and the data processing module is achieved, and the asynchronous processing between the data access module and the data processing module is facilitated. Furthermore, through the first message platform, data peak reduction and flow control can also be achieved, and the data is buffered to avoid network transmission congestion due to the large amount of data.

The data storage module 203 is configured to store the medical data of the subject to be treated. In some embodiments, the data storage module 203 may further be configured to store the standard indicator and the corresponding indicator information.

In embodiments of the present disclosure, the data storage module 203 may use the relational database such as MariaDB, MySQL, etc., the document database such as MongoDB, CouchDB, etc., the distributed file system such as HDFS, PVFS, PanFS, etc., and the graph database such as Neo4j, Cayley, rapgDB, etc. In addition, some data may be written into a distributed cache such as Redis, Memcached, SSDB, etc. according to a data call frequency and a data type, thereby improving a reading speed.

The data distribution module 204 is configured to distribute the medical data of the subject to be treated processed by the data processing module to the medical business system.

In embodiments of the present disclosure, a second message platform 206 is further configured between the data distribution module 204 and the medical business system 103. The second message platform 206 is used by the data distribution module 204 to transmit the medical data of the subject to be treated to the medical business system 103 in the message service manner. Then, the data distribution module 204 writes the medical data of the subject to be treated into the message queue such as RabbitMQ, ActiveMQ, ZeroMQ, Kafka, MetaMQ, RocketMQ, etc. The medical business system 103 may pull data from the message queue in the first-in-first-out manner for processing. By configuring the second message platform between the data distribution module and the medical business system, decoupling between the data distribution module and the medical business system is achieved, and the asynchronous processing between the data distribution module and the medical business system is facilitated. Furthermore, through the second message platform, data peak reduction and flow control can also be achieved, and the data is buffered to avoid network transmission congestion due to the large amount of data.

In addition, in some embodiments of the present disclosure, the data distribution module may further be configured to provide the medical data of the subject to be treated to the second message platform 206 in a HTTP transmission manner, a RSA encrypted transmission manner, a message queue manner, etc. For example, the data distribution module 204 writes the medical data of the subject to be treated into the message queue to facilitate the second message platform 206 to pull the medical data of the subject to be treated from the message queue.

In some embodiments, specific positions for providing the first message platform and the second message platform are not limited. For example, they may also be provided at other positions other than the position between the data access module and the data processing module and the position between the data distribution module and the medical business system.

In some implementations, the first message platform and the second message platform may be implemented by the same message platform. There is no limitation on the number of message platforms.

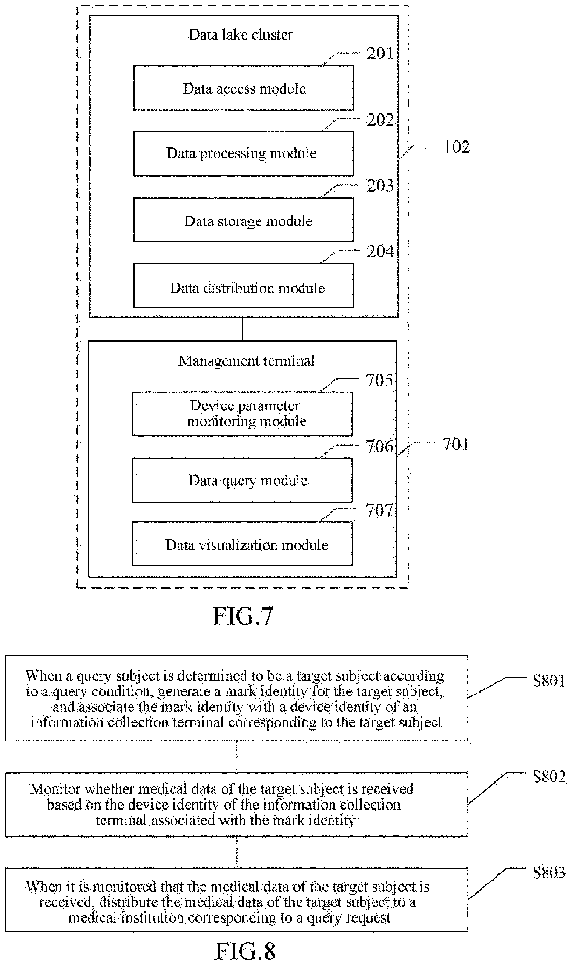

Referring to FIG. 7, in some embodiments of the present disclosure, the emergency treatment system may further include a management terminal 701.

In some embodiments, the management terminal 701 includes a device parameter monitoring module 705. The device parameter monitoring module 705 is configured to record and analyze behavior information of the information collection terminal to determine whether the information collection terminal is an abnormal terminal. In an embodiment of the present disclosure, the behavior information of the information collection terminal may include a usage time, the number of exceptions, etc. For example, the device parameter monitoring module 705 may record behavior information of a normal information collection terminal, such as the usage time, the number of exceptions, etc., and perform statistical analysis based on this to determine a fluctuation range of the behavior information of the normal information collection terminal, such as the usage time, the number of exceptions, etc. As a result, when it is monitored that behavior information of a certain information collection terminal, such as the usage time, the number of exceptions, etc., exceeds the above fluctuation range, this information collection terminal may be considered as the abnormal terminal.

In other embodiments of the present disclosure, sample data may also be obtained based on historical behavior information of the information collection terminal, so that one or more machine learning models, such as a random forest model, a deep neural network model, a support vector machine model, a boosted tree model, a general linear model and a progressive gradient regression tree model, are trained based on the sample data, so as to obtain an abnormal terminal determination model. In turn, the device parameter monitoring module 705 may input behavior information of a certain information collection terminal into the abnormal terminal determination model, so as to output, by means of the abnormal terminal determination model, a probability that the information collection terminal is the abnormal terminal. When a probability value that the information collection terminal is the abnormal terminal exceeds a threshold value, the information collection terminal may be considered to be the abnormal terminal.

In some embodiments of the present disclosure, the management terminal may further be configured to record the device identity and a device start of use time of the information collection terminal. In some embodiments, the management terminal may further record an end of use time;

receive the medical data of the subject to be treated distributed from the data lake cluster; and
associate the medical data with user information of the subject to be treated based on the device identity and the device start of use time.

The medical business system is further configured to receive the medical data associated with the user information of the subject to be treated.

The process of recording the device identity, the device start of use time and the end of use time of the information collection terminal may be completed by the data processing module in the data lake cluster and then sent to the management terminal, or the management terminal may directly complete the above recording process.

For example, information obtained after the medical data collected by the information collection terminal is associated with the device identity, the device start of use time and the end of use time is as shown in Table 3 below:

Table 3

| Device Identity | Standard indicator code | Indicator value | time |
|---|---|---|---|
| 2820 | 291 | 0.00 | 2021-03-10 05:36:01 |
| 2820 | 291 | 1.00 | 2021-03-10 05:35:01 |
| 2820 | 291 | 1.00 | 2021-03-10 05:34:01 |
| 2820 | 291 | 0.00 | 2021-03-10 05:33:01 |
| 2820 | 291 | 2.00 | 2021-03-10 05:32:01 |
| 2820 | 291 | 0.00 | 2021-03-10 05:31:01 |
| 2820 | 291 | 1.00 | 2021-03-10 05:30:01 |
| 2820 | 291 | 0.00 | 2021-03-10 05:29:01 |
| 2820 | 291 | 0.00 | 2021-03-10 05:28:01 |
| 2820 | 291 | 0.00 | 2021-03-10 05:27:01 |
| 2820 | 291 | 0.00 | 2021-03-10 05:26:01 |
| 2820 | 291 | 0.00 | 2021-03-10 05:25:01 |
| 2820 | 291 | 0.00 | 2021-03-10 05:24:01 |
| 2820 | 291 | 1.00 | 2021-03-10 05:23:01 |
| 2820 | 291 | 0.00 | 2021-03-10 05:22:01 |

The management terminal receives the medical data distributed from the data lake cluster, and the medical data at least includes the device identity, the device start of use time and the indicator information.

In some embodiments, the user information includes an identity of a user of the information collection terminal, which may include, for example, at least one of name, ID number, etc. Since data stored in the data lake cluster does not include the user information of the user, which may play a role of desensitizing the user information. When relevant medical data of the user is sent to the medical business system, the user information needs to be associated with the medical data.

In some embodiments, the user information may be obtained when the user makes an alarm through an alarm terminal and/or user's personal information is entered through a doctor terminal in the ambulance vehicle, and a time for making the alarm and/or entering the personal information on the doctor terminal may also be recorded at the same time. The user's user information and the recording time may be sent to the management terminal. According to the device identity and the device start of use time, the time corresponds to the user that obtains the personal information, thereby completing the association of the medical data with the user information of the subject to be treated. The medical data associated with the user information of the subject to be treated (that is, the medical data carrying the user information) is sent to the medical business system.

For example, the management terminal obtains: the user identity UserID: 123321; the device identity SN: 2820; the device start of use time BindingTimeStart: 2021-03-10 05:22:01; the device end of use time BindingTimeEnd: 2021-03-10 05:36:01.

In turn, after obtaining the information in Table 3 sent by the data lake cluster, the management terminal may, according to the device identity, the device start of use time and the end of use time in Table 3, perform matching for a time when the management terminal obtains the user information to obtain the user identity for the information in Table 3, and bind the data in Table 3 with the user identity. Therefore, through the above method, the data lake cluster does not need to store the user identity information, but only stores the device information and the medical data, thereby realizing the function of desensitizing the user identity information.

In some embodiments of the present disclosure, the management terminal may further be configured to store historical medical data of the subject to be treated, and jointly distribute the historical medical data and real-time medical data of the subject to be treated to the medical business system. Specifically, the management terminal may obtain the user identity corresponding to the device identity of the information collection terminal; thus, it may also obtain the historical medical data of the subject to be treated corresponding to the user identity through the user identity, and jointly distribute the historical medical data and the real-time medical data of the subject to be treated to the medical business system, which enables a user of the medical business system (such as a medical worker) to more comprehensively understand information of the subject to be treated.

In some embodiments of the present disclosure, the management terminal may further be configured to store medical data of relatives and friends of the subject to be treated, and jointly distribute the medical data of the relatives and friends of the subject to be treated and the medical data of the subject to be treated to the medical business system. Specifically, the management terminal stores the user identity corresponding to the device identity of the information collection terminal. Therefore, an identity of a user who has a relationship of relatives and friends with the user may also be obtained through the identity of the user of the current information collection terminal. Then, the medical data of the relatives and friends of the subject to be treated is obtained in the management terminal through the identity of the relatives and friends, and the medical data of the relatives and friends of the subject to be treated and the medical data of the subject to be treated are jointly distributed to the medical business system, thereby enabling the user of the medical business system (such as the medical worker) to understand genetic disease information or infectious disease information of the subject to be treated, etc.

With the continued reference to FIG. 7, in an embodiment of the present disclosure, the management terminal 701 may further include a data query module 706. The data query module 706 may be configured to receive a query request, and obtain, according to a query condition included in the query request, relevant data from the data lake cluster to generate a query result. For example, in this embodiment of the present disclosure, after the query request of the medical institution is received, a query field included in the query request is obtained, and an operation, such as equal to, greater than, less than, equal to or greater than, equal to or less than, not equal to, etc. is performed in the data lake cluster on the query field according to the query request, so as to achieve a precise query. Alternatively, a fuzzy query may also be realized according to the query field by using a LIKE mechanism or a regular expression of a SQL statement, which is not particularly limited in embodiments of the present disclosure.

Referring to FIG. 8, in some embodiments of the present disclosure, the data query module 706 may further be configured to perform steps S801 to S803.

In the step S801, when a query subject is determined to be a target subject according to a query condition, a mark identity is generated for the target subject, and the mark identity is associated with a device identity of an information collection terminal corresponding to the target subject.

For example, in an embodiment of the present disclosure, if a medical institution needs to obtain medical data of the target subject who meets the following query condition: in a specified area, gender is female, age is greater than 50 years old, the blood diastolic blood pressure is greater than 90mmHg, and the blood systolic blood pressure is greater than 140mmHg, the data query module may determine whether the query subject satisfies the above query condition. If the query condition is satisfied, the query subject will be regarded as the target subject. Then the mark identity may be generated for the information collection terminal corresponding to the target subject, for example, an attribute identity of Select=1 is generated to mark the query subject as the target subject. Correspondingly, if the query condition is not satisfied, an attribute identity of Select=0 may be generated to mark the query subject as not the target subject.

In the step S802, it is monitored whether medical data of the target subject is received based on the device identity of the information collection terminal associated with the mark identity.

For example, the data query module generates, according to the mark identity, a mark identity generation time and the device identity corresponding to the target subject, a monitoring rule "device identity SN: A123456, mark identity generation time Binding_Time: 2020-09-08-12:12:12, mark identity Select: 1, diastolic blood pressure: greater than 90mmHg, systolic blood pressure: greater than 140mmHg", and determines whether an information collection terminal corresponding to newly accessed medical data satisfies the monitoring rule; and if monitoring rule is satisfied, determines that the medical data of the target subject has been received.

In the step S803, when it is monitored that the medical data of the target subject is received, the medical data of the target subject is distributed to the medical institution corresponding to the query request.

With the continued reference to FIG. 7, in some embodiments of the present disclosure, the management terminal 701 may further include a data visualization module 707. The data visualization module 707 may be configured to generate visual content according to data of the data lake cluster to display the visual content through a front-end page.

In some implementations, the processes in the data lake cluster such as the data access, the data processing, the data distribution may be displayed to the user by the data visualization module 707, so as to facilitate the user to monitor each process. Moreover, the stored medical data may also be displayed by the data visualization module 707 .

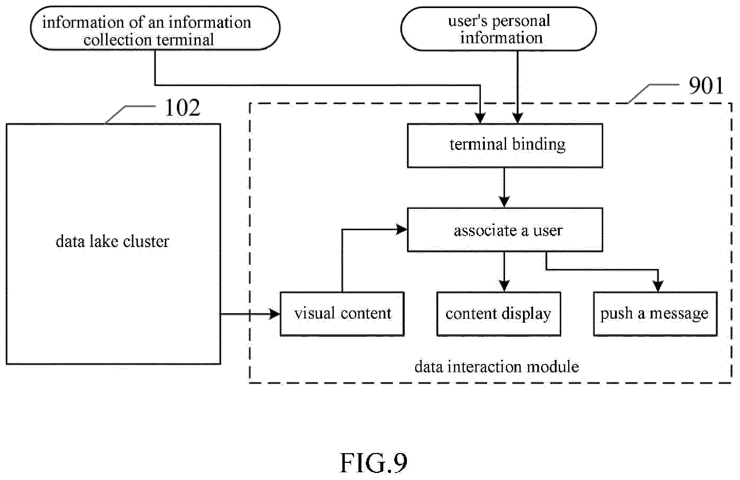

For example, referring to FIG. 9, in an embodiment, the management terminal and/or the medical business system may also be configured with a data interaction module 901. The data interaction module 901 can obtain the user's personal information and the information of the information collection terminal corresponding to the user and perform terminal binding. The user's personal information may include a region where the user is located, age, gender, etc. The information of the information collection terminal may include manufacturer information, the device identity, device system information, the indicator information and analyzing dictionary information, etc.

In some implementations, the data interaction module 901 may send a request to the data visualization module 707 of the management terminal, so as to facilitate the data visualization module 707 to generate the visual content according to the data in the data lake cluster. In turn, after receiving the visual content, the data interaction module 901 may display it to the user through the front-end page. In addition, in some embodiments, the data interaction module 901 may also actively push a message to the user, such as a device usage reminder message, a health reminder message, etc.

The medical business system 203 can obtain the medical data of the subject to be treated from the data lake cluster.

In some embodiments of the present disclosure, there is a data conversion relationship between the medical data of the subject to be treated accessed by the data access module and the processed medical data of the subject to be treated distributed by the data distribution module. In some implementations, an input indicator includes a hypopnea index $x1$, an apnea index $x2$ and a pressure parameter $x3$, an output indicator includes a sleep apnea hypopnea index mean $y1$, and the following data conversion relationship exists between $y1$ and $x1$, $x2$ and $x3$:

$y1=f1(x1,x2,x3)$; where $f1$ represents a linear conversion relationship or a nonlinear conversion relationship.

For example, the data conversion relationship is: $y1=w1*x1+w2*x2+w3*x3$; where $w1$, $w2$, and $w3$ are relational parameters.

For example, a value range of $w1$ is [1,1.00000011], a value range of $w2$ is [1,1.00000011], and a value range of $w3$ is [7.85046229E-18, 7.85046229E-16].

For example, a value of $w1$ is 1.00000011, a value of $w2$ is 1.00000011, and a value of $w3$ is 7.85046229E-17.

In some embodiments, the input indicator includes the hypopnea index $x1$, the apnea index $x2$ and the pressure parameter $x3$, the output indicator is an apnea index mean $y2$, and the following data conversion relationship exists between $y2$ and $x1$, $x2$ and $x3$:

$y2=f2(x1,x2,x3)$; where $f2$ represents the linear conversion relationship or the nonlinear conversion relationship.

For example, the data conversion relationship is: $y2=w1*xl+w2*x2+w3*x3$; where $w1$, $w2$, and $w3$ are relational parameters.

For example, the value range of $w1$ is [5.55500801E-08, 0.50000006], the value range of $w2$ is [0.50000006, 1.00000006], and the value range of $w3$ is [2.22044605E-17, 2.22044605E-15].

For example, the value of $w1$ is 0.50000006, 0.500000056 or 5.55500801E-08, the value of $w2$ is 0.50000006 or 1.00000006, and the value of $w3$ is 2.22044605E-16.

In some embodiments, the input indicator includes the hypopnea index $x1$, the apnea index $x2$ and the pressure parameter $x3$, the output indicator includes a hypopnea index mean $y3$, and the following data conversion relationship exists between $y3$ and $x1$, $x2$ and $x3$:

$y3=f3(x1,x2,x3)$; where $f3$ represents the linear conversion relationship or the nonlinear conversion relationship.

For example, the data conversion relationship is: $y3=w1*x1+w2*x2+w3*x3$; where $w1$, $w2$ and $w3$ are relational parameters.

For example, the value range of $w1$ is [0.500000056, 1.00000006], the value range of $w2$ is [5.55500802E-08, 0.50000006], and the value range of $w3$ is [-7.85046229E-16, -7.85046229E -18].

For example, the value of $w1$ is 0.50000006, 0.500000056 or 1.00000006, the value of $w2$ is 5.55500802E-08 or 0.50000006, and the value of w3 is -7.85046229E-17.

In some embodiments of the present disclosure, the data conversion relationship between the medical data of the subject to be treated accessed by the data access module and the processed medical data of the subject to be treated distributed by the data distribution module is obtained in the following manner.

The machine learning model may be trained using sample medical data and sample business data to obtain the data conversion relationship. For example, the data conversion relationship may be obtained through a neural network model, such as a convolutional neural network model, a residual neural network model, a recurrent neural network model, a long short-term memory neural network model, etc. The data conversion relationship may also be obtained through a collaborative filtering model, a hidden Markov model, a conditional random field model, etc.

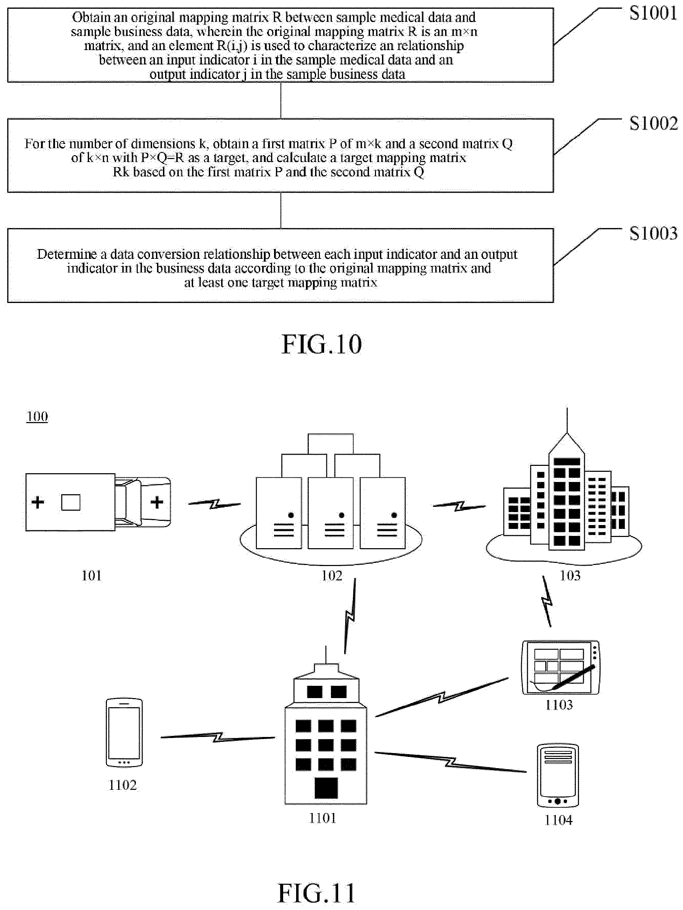

Referring to FIG. 10, in an embodiment of the present disclosure, the process of obtaining the data conversion relationship may include steps S1001 to S1003.

In the step S1001, an original mapping matrix R between the sample medical data and the sample business data is obtained. The original mapping matrix R is an $m \times n$ matrix, and an element $R(i,j)$ is used to characterize an relationship between an input indicator i in the sample medical data and an output indicator j in the sample business data. M and n are integers greater than 1, i, j, and k are positive integers, and $i \in [1,m]$, $j \in [1,n]$, $k \in [1,m-1]$.

For example, sample medical data uploaded to the data lake cluster by an information collection terminal includes 9 input indicators, and sample business data generated by the data lake cluster based on medical data provided by an information collection terminal includes 10 output indicators. An original mapping matrix R between the sample medical data and the sample business data is for example shown in Table 4 below.

Table 4

|  | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|
| IN-01 | 1 | 0 | 0 | 0 | 0 | 0 | 0 | 0 | 0 | 1 |
| IN-02 | 0 | 0 | 1 | 0 | 0 | 0 | 0 | 0 | 0 | 0 |
| IN-03 | 0 | 0 | 0 | 0 | 0 | 0 | 0 | 1 | 0 | 0 |
| IN-04 | 0 | 0 | 0 | 0 | 1 | 0 | 0 | 0 | 0 | 0 |
| IN-05 | 0 | 0 | 0 | 0 | 0 | 0 | 1 | 0 | 0 | 0 |
| IN-06 | 1 | 0 | 0 | 0 | 0 | 0 | 0 | 0 | 1 | 0 |
| IN-07 | 0 | 1 | 0 | 0 | 0 | 0 | 0 | 0 | 0 | 0 |
| IN-08 | 0 | 0 | 0 | 0 | 0 | 1 | 0 | 0 | 0 | 0 |
| IN-09 | 0 | 0 | 0 | 1 | 0 | 0 | 0 | 0 | 0 | 0 |

The original mapping matrix R is a $9 \times 10$ matrix, and the element $R(i,j)$ is used to characterize the relationship between the input indicator i in the sample medical data and the output indicator j in the sample business data. For example, a value of the element $R(1,1)$ is 1, which means that there is a correlation between the input indicator IN-01 and the output indicator OUT-01. A value of element $R(1,2)$ is 0, which means that whether there is a correlation between the input indicator IN-01 and the output indicator OUT-01 cannot be determined. Taking the output indicator OUT-01 as the sleep apnea hypopnea index mean, IN-01 as the hypopnea index, and IN-06 as the apnea index as an example, it is currently known that the sleep apnea hypopnea index mean is related to the hypopnea index and the apnea index. For example, the sleep apnea hypopnea index mean is the sum of the hypopnea index and the apnea index, and thus, values of $R(1,1)$ and $R(6,1)$ are 1. However, whether there are correlations between the sleep apnea hypopnea index and other input indicators cannot be determined at present (that is, there may or may not be the correlation), and thus, values of $R(2,1)$, $R(3,1)$, etc. are all 0.

In the step S1002, for the number of dimensions k, a first matrix P of $m \times k$ and a second matrix Q of $k \times n$ are obtained with $P \times Q = R$ as a target, and a target mapping matrix $R_k$ is calculated based on the first matrix P and the second matrix Q, where k is a positive integer, and $k \in [1,m-1]$.

In embodiments of the present disclosure, hidden information mining may be performed based on the original mapping matrix to obtain the first matrix and the second matrix, so that the first matrix represents a degree of influence of the input indicator on each output indicator, and the second matrix represents a degree of the output indicator being influenced by each input indicator. Then, a third matrix is obtained by multiplying the first matrix and the second matrix, which includes a correlation between each input indicator and each output indicator. That is, for the element in the above Table 4 where it is impossible to determine whether there is the correlation between the input indicator and the output indicator, whether there is the correlation between the input indicator and the output indicator can be determined through the third matrix.

In some implementations, a first matrix P of m×m, a second matrix Q of n×n, and an intermediate matrix S of m × n may also be obtained with R=P×S×Q as the target, and the target mapping matrix $R_k$ is calculated based on the first matrix P, the second matrix Q and the intermediate matrix S.

In some implementations, the first matrix P of m×m, a second matrix Q of m×n, and an intermediate matrix S of m× m may also be obtained with R=P×S×Q as the target, and the target mapping matrix $R_k$ is calculated based on the first matrix P, the second matrix Q and the intermediate matrix S.

Specific dimensions of matrices P, S, and Q may be adjusted as needed, which are not specifically limited here.

Taking the original mapping matrix R in the above Table 4 as an example, in an embodiment of the present disclosure, singular value decomposition may be first performed on the original mapping matrix R to obtain a singular value Σ[1.73205081,1,1,1, 1,1,1,1,1] of the original mapping matrix R, and the first matrix P and the second matrix Q may be obtained at the same time, for example, the first matrix P of 9×9 and the second matrix Q of 10×10 are obtained.

For example, when the number of dimensions k is 8, the intermediate matrix S is constructed through the first 8 values of the singular value Σ. The intermediate matrix S is a 9×10 matrix as shown in Table 5 below:

Table 5

| 1.73205081 | 0 | 0 | 0 | 0 | 0 | 0 | 0 | 0 | 0 |
|---|---|---|---|---|---|---|---|---|---|
| 0 | 1 | 0 | 0 | 0 | 0 | 0 | 0 | 0 | 0 |
| 0 | 0 | 1 | 0 | 0 | 0 | 0 | 0 | 0 | 0 |
| 0 | 0 | 0 | 1 | 0 | 0 | 0 | 0 | 0 | 0 |
| 0 | 0 | 0 | 0 | 1 | 0 | 0 | 0 | 0 | 0 |
| 0 | 0 | 0 | 0 | 0 | 1 | 0 | 0 | 0 | 0 |
| 0 | 0 | 0 | 0 | 0 | 0 | 1 | 0 | 0 | 0 |
| 0 | 0 | 0 | 0 | 0 | 0 | 0 | 1 | 0 | 0 |
| 0 | 0 | 0 | 0 | 0 | 0 | 0 | 0 | 0 | 0 |

Specifically, the original mapping matrix R may be decomposed to obtain the first matrix P and the second matrix Q. For example, Python is used to call a function such as linalg.svd to decompose the original mapping matrix R, or the original mapping matrix R is decomposed in other ways. In some implementations, the singular value Σ may also be obtained.

In some implementations, after the first matrix P and the second matrix Q are obtained, and the target mapping matrix $R_k$ is calculated according to the product of the first matrix P and the second matrix Q.

In some embodiments, the target mapping matrix $R_k$ is calculated according to the product of the first matrix P, the second matrix Q, and the intermediate matrix S.

The resulting target mapping matrix $R_k$ is shown in Table 6 below:

Table 6

| | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|
| IN-01 | 1.00000011 | 0 | 0 | 0 | 0 | 0 | 0 | 0 | 5.55500801E-08 | 1.00000006 |
| IN-02 | 0 | 0 | 1 | 0 | 0 | 0 | 0 | 0 | 0 | 0 |
| IN-03 | 0 | 0 | 0 | 0 | 0 | 0 | 0 | 1 | 0 | 0 |
| IN-04 | 0 | 0 | 0 | 0 | 1 | 0 | 0 | 0 | 0 | 0 |
| IN-05 | 0 | 0 | 0 | 0 | 0 | 0 | 1 | 0 | 0 | 0 |
| IN-06 | 1.00000011 | 0 | 0 | 0 | 0 | 0 | 0 | 0 | 1.00000006 | 5.55500802E-08 |
| IN-07 | 7.85046229E-17 | 1 | 0 | 0 | 0 | 0 | 0 | 0 | 2.22044605E-16 | -7.85046229E-17 |
| IN-08 | 0 | 0 | 0 | 0 | 0 | 1 | 0 | 0 | 0 | 0 |
| IN-09 | 0 | 0 | 0 | 1 | 0 | 0 | 0 | 0 | 0 | 0 |

EP 4 345 840 A1

Similarly, when the number of dimensions k is 5, the intermediate matrix S is constructed through the first five values of the singular value $\Sigma$. The intermediate matrix S is a $9\times10$ matrix as shown in Table 7 below:

Table 7

| 1.53205051 | 0 | 0 | 0 | 0 | 0 | 0 | 0 | 0 | 0 |
|---|---|---|---|---|---|---|---|---|---|
| 0 | 1 | 0 | 0 | 0 | 0 | 0 | 0 | 0 | 0 |
| 0 | 0 | 1 | 0 | 0 | 0 | 0 | 0 | 0 | 0 |
| 0 | 0 | 0 | 1 | 0 | 0 | 0 | 0 | 0 | 0 |
| 0 | 0 | 0 | 0 | 1 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |

Specifically, the original mapping matrix R may be decomposed to obtain the first matrix P and the second matrix Q. For example, Python is used to call a function such as linalg.svd to decompose the original mapping matrix R, or the original mapping matrix R is decomposed in other ways. In some implementations, the singular value $\Sigma$ may also be obtained.

In some implementations, after the first matrix P and the second matrix Q are obtained, and the target mapping matrix $R_k$ is calculated according to the product of the first matrix P and the second matrix Q.

In some embodiments, the target mapping matrix $R_k$ is calculated according to the product of the first matrix P, the second matrix Q, and the intermediate matrix S.

The resulting target mapping matrix $R_k$ is shown in Table 8 below:

Table 8

| | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|
| IN-01 | 1 | 0 | 0 | 0 | 0 | 0 | 0 | 0 | 5.55500801E-08 | 1.00000006 |
| IN-02 | 0 | 0 | 1 | 0 | 0 | 0 | 0 | 0 | 0 | 0 |
| IN-03 | 0 | 0 | 0 | 0 | 0 | 0 | 0 | 1 | 0 | 0 |
| IN-04 | 0 | 0 | 0 | 0 | 1 | 0 | 0 | 0 | 0 | 0 |
| IN-05 | 0 | 0 | 0 | 0 | 0 | 0 | 1 | 0 | 0 | 0 |
| IN-06 | 1 | 0 | 0 | 0 | 0 | 0 | 0 | 0 | 1.00000006 | 5.55500802E-08 |
| IN-07 | 7.85046229E-17 | 1 | 0 | 0 | 0 | 0 | 0 | 0 | 2.22044605E-16 | -7.85046229E-17 |
| IN-08 | 0 | 0 | 0 | 0 | 0 | 1 | 0 | 0 | 0 | 0 |
| IN-09 | 0 | 0 | 0 | 1 | 0 | 0 | 0 | 0 | 0 | 0 |

Similarly, when the number of dimensions k is 1, the intermediate matrix S is constructed through the first value of the singular value $\Sigma$. The intermediate matrix S is a $9 \times 10$ matrix as shown in Table 9 below:

Table 9

| 1.53205051 | 0 | 0 | 0 | 0 | 0 | 0 | 0 | 0 | 0 |
|---|---|---|---|---|---|---|---|---|---|
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |
| 0 | 0 | 0 | 0 | 0 | 0 | 0 | 0 | 0 | 0 |

Specifically, the original mapping matrix R may be decomposed to obtain the first matrix P and the second matrix Q. For example, Python is used to call a function such as linalg.svd to decompose the original mapping matrix R, or the original mapping matrix R is decomposed in other ways. In some implementations, the singular value $\Sigma$ may also be obtained.

In some implementations, after the first matrix P and the second matrix Q are obtained, and the target mapping matrix $R_k$ is calculated according to the product of the first matrix P and the second matrix Q.

In some embodiments, the target mapping matrix $R_k$ is calculated according to the product of the first matrix P, the second matrix Q, and the intermediate matrix S.

The resulting target mapping matrix $R_k$ is shown in Table 10 below:

Table 10

| | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|
| IN-01 | 1.00000011 | 0 | 0 | 0 | 0 | 0 | 0 | 0 | 0.50000006 | 0.50000006 |
| IN-02 | 0 | 0 | 1 | 0 | 0 | 0 | 0 | 0 | 0 | 0 |
| IN-03 | 0 | 0 | 0 | 0 | 0 | 0 | 0 | 1 | 0 | 0 |
| IN-04 | 0 | 0 | 0 | 0 | 1 | 0 | 0 | 0 | 0 | 0 |
| IN-05 | 0 | 0 | 0 | 0 | 0 | 0 | 1 | 0 | 0 | 0 |
| IN-06 | 1.00000011 | 0 | 0 | 0 | 0 | 0 | 0 | 0 | 0.50000006 | 0.50000006 |
| IN-07 | 0 | 1 | 0 | 0 | 0 | 0 | 0 | 0 | 0 | 0 |
| IN-08 | 0 | 0 | 0 | 0 | 0 | 1 | 0 | 0 | 0 | 0 |
| IN-09 | 0 | 0 | 0 | 1 | 0 | 0 | 0 | 0 | 0 | 0 |

In other embodiments of the present disclosure, the first matrix and second matrix may also be determined in other ways. For example, in an embodiment of the present disclosure, the first matrix and the second matrix may also be determined through a gradient descent algorithm combined with a preset loss function. In more embodiments of the present disclosure, it is also possible to determine the first matrix and the second matrix through the neural network model, which is not particularly limited in embodiments of the present disclosure.

In the step S 1003, a data conversion relationship between each input indicator and an output indicator in the business data is determined according to the original mapping matrix and at least one target mapping matrix.

In embodiments of the present disclosure, the data conversion relationship between each input indicator and the output indicator in the business data may be determined based on the original mapping matrix and the 1st to (n-1)-th target mapping matrices. Alternatively, in other embodiments of the present disclosure, in order to reduce the amount of calculations, the data conversion relationship may also be determined according to the original mapping matrix and a smaller number of target mapping matrices(for example, the 2nd to (n-2)-th target mapping matrices, target mapping matrices with odd sequence numbers, target mapping matrices with even sequence number, etc.), which is not particularly limited in embodiments of the present disclosure.

Taking the determination of the data conversion relationship based on the original mapping matrix and the 1st to 8th target mapping matrices as an example, parameters in the 1st to 8th target mapping matrices may be summarized to obtain Table 11 below.

Table 11

| | k | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|---|
| IN-01 | 1 | 1.00000011 | | | | | | | | 0.50000006 | 0.50000006 |
| | 2 | 1.00000011 | | | | | | | | 0.500000056 | 0.500000056 |
| | 3 | 1.00000011 | | | | | | | | 5.55500801E-08 | 1.00000006 |
| | 4 | 1.00000011 | | | | | | | | 5.55500801E-08 | 1.00000006 |
| | 5 | 1 | | | | | | | | 5.55500801E-08 | 1.00000006 |
| | 6 | 1.00000011 | | | | | | | | 5.55500801E-08 | 1.00000006 |
| | 7 | 1.00000011 | | | | | | | | 5.55500801E-08 | 1.00000006 |
| | 8 | 1.00000011 | | | | | | | | 5.55500801E-08 | 1.00000006 |
| IN-02 | | | | 1 | | | | | | | |
| IN-03 | | | | | | | | 1 | | | |
| IN-04 | | | | | | 1 | | | | | |
| IN-05 | | | | | | | | 1 | | | |
| IN-06 | 1 | 1.00000011 | | | | | | | | 0.50000006 | 0.50000006 |
| | 2 | 1.00000011 | | | | | | | | 0.50000006 | 0.50000006 |
| | 3 | 1.00000011 | | | | | | | | 1.00000006 | 5.55500802E-08 |
| | 4 | 1.00000011 | | | | | | | | 1.00000006 | 5.55500802E-08 |
| | 5 | 1 | | | | | | | | 1.00000006 | 5.55500802E-08 |
| | 6 | 1.00000011 | | | | | | | | 1.00000006 | 5.55500802E-08 |
| | 7 | 1.00000011 | | | | | | | | 1.00000006 | 5.55500802E-08 |
| | 8 | 1.00000011 | | | | | | | | 1.00000006 | 5.55500802E-08 |

(continued)

| | k | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|---|
| IN-07 | 1 | | 1 | | | | | | | | |
| | 2 | 7.85046229E-17 | 1 | | | | | | | 2.22044605E-16 | -7.85046229E-17 |
| | 3 | 7.85046229E-17 | 1 | | | | | | | 2.22044605E-16 | -7.85046229E-17 |
| | 4 | 7.85046229E-17 | 1 | | | | | | | 2.22044605E-16 | -7.85046229E-17 |
| | 5 | 7.85046229E-17 | 1 | | | | | | | 2.22044605E-16 | -7.85046229E-17 |
| | 6 | 7.85046229E-17 | 1 | | | | | | | 2.22044605E-16 | -7.85046229E-17 |
| | 7 | 7.85046229E-17 | 1 | | | | | | | 2.22044605E-16 | -7.85046229E-17 |
| | 8 | 7.85046229E-17 | 1 | | | | | | | 2.22044605E-16 | -7.85046229E-17 |
| IN-08 | | | | | | | 1 | | | | |
| IN-09 | | | | | 1 | | | | | | |

Data that does not need to be processed in the Table 11 is removed to obtain Table 12 below.

Table 12

| | k | OUT-01 | OUT-02 | OUT-03 | OUT-04 | OUT-05 | OUT-06 | OUT-07 | OUT-08 | OUT-09 | OUT-10 |
|---|---|---|---|---|---|---|---|---|---|---|---|
| IN-01 | 1 | 1.00000011 | | | | | | | | 0.50000006 | 0.50000006 |
| | 2 | 1.00000011 | | | | | | | | 0.500000056 | 0.500000056 |
| | 3 | 1.00000011 | | | | | | | | 5.55500801E-08 | 1.00000006 |
| | 4 | 1.00000011 | | | | | | | | 5.55500801E-08 | 1.00000006 |
| | 5 | 1 | | | | | | | | 5.55500801E-08 | 1.00000006 |
| | 6 | 1.00000011 | | | | | | | | 5.55500801E-08 | 1.00000006 |
| | 7 | 1.00000011 | | | | | | | | 5.55500801E-08 | 1.00000006 |
| | 8 | 1.00000011 | | | | | | | | 5.55500801E-08 | 1.00000006 |
| IN-06 | 1 | 1.00000011 | | | | | | | | 0.50000006 | 0.50000006 |
| | 2 | 1.00000011 | | | | | | | | 0.50000006 | 0.50000006 |
| | 3 | 1.00000011 | | | | | | | | 1.00000006 | 5.55500802E-08 |
| | 4 | 1.00000011 | | | | | | | | 1.00000006 | 5.55500802E-08 |
| | 5 | 1 | | | | | | | | 1.00000006 | 5.55500802E-08 |
| | 6 | 1.00000011 | | | | | | | | 1.00000006 | 5.55500802E-08 |
| | 7 | 1.00000011 | | | | | | | | 1.00000006 | 5.55500802E-08 |
| | 8 | 1.00000011 | | | | | | | | 1.00000006 | 5.55500802E-08 |
| IN-07 | 1 | 7.85046229E-17 | 1 | | | | | | | | |
| | 2 | 7.85046229E-17 | 1 | | | | | | | 2.22044605E-16 | -7.85046229E-17 |
| | 3 | 7.85046229E-17 | 1 | | | | | | | 2.22044605E-16 | -7.85046229E-17 |
| | 4 | 7.85046229E-17 | 1 | | | | | | | 2.22044605E-16 | -7.85046229E-17 |
| | 5 | 7.85046229E-17 | 1 | | | | | | | 2.22044605E-16 | -7.85046229E-17 |
| | 6 | 7.85046229E-17 | 1 | | | | | | | 2.22044605E-16 | -7.85046229E-17 |
| | 7 | 7.85046229E-17 | 1 | | | | | | | 2.22044605E-16 | -7.85046229E-17 |
| | 8 | 7.85046229E-17 | 1 | | | | | | | 2.22044605E-16 | -7.85046229E-17 |

In embodiments of the present disclosure, the data conversion relationship may be:

$$y_j = f(x_i, i \in [1, m])$$

where, $y_j$ is a value of an output indicator j, a parameter of the function $f$ is determined based on the original mapping matrix and element values of a column where the output indicator j is located in at least one target mapping matrix, and xi is a value of an input indicator i. For example, for a value of an output indicator $y_1$ (that is, OUT-01) in the above Table 12, its data conversion relationship with values of an input indicator $x_1$ (that is, IN-01), an input indicator $x_2$ (that is, IN-06), and an input indicator $x_3$ (that is, IN-07) is: $y_1 = f(x_1, x_2, x_3)$. Similarly, the correlation between other input indicators and output indicators may be determined. A method for determining the parameter of the function $f$ will be explained below with reference to examples.

For example, the input indicator IN-01 is the hypopnea index $x_1$, the input indicator IN-06 is the apnea index $x_2$, and the input indicator IN-07 is the pressure parameter $x_3$, the output indicator OUT-01 is the sleep apnea hypopnea index mean $y_1$, and a conversion mathematical relationship corresponding to the sleep apnea hypopnea index mean is:

$$y_1 = f_1(x_1, x_2, x_3)$$

where, a parameter of the function $f_1$ is determined based on the original mapping matrix and element values of a column (i.e., column 1) where the sleep apnea hypopnea index mean is located in at least one target mapping matrix (the 1st to 8th target mapping matrices are taken as an example here). For example, a relationship between the sleep apnea hypopnea index mean and the hypopnea index, the apnea index and the pressure parameter is a weighted sum, and the corresponding conversion relationship may be specifically:

$$y_1 = w_1 * x_1 + w_2 * x_2 + w_3 * x_3$$

where, $w_1$, $w_2$ and $w_3$ are determined based on the original mapping matrix and element values of a column where the sleep apnea hypopnea index mean is located in at least one target mapping matrix. For example, in the above Table 12, element values in a first column of a row where the input indicator IN-01 is located are respectively: 1.00000011, 1.00000011, 1.00000011, 1.00000011, 1, 1.00000011, 1.00000011, 1.00000011, and then a value range of $w_1$ may be [1, 1.00000011]. Similarly, it may be determined that a value range of $w_2$ is [1, 1.00000011], and a value range of $w_3$ is [7.85046229E-18, 7.85046229E-16].

As verified by the inventor, in order to achieve a more accurate calculation result, in the conversion mathematical relationship corresponding to the sleep apnea hypopnea index mean, a value of $w_1$ may be specifically 1.00000011, a value of $w_2$ may be specifically 1.00000011, and a value of $w_3$ may be specifically 7.85046229E-17.

For another example, the input indicator IN-01 is the hypopnea index $x_1$, the input indicator IN-06 is the apnea index $x_2$, and the input indicator IN-07 is the pressure parameter $x_3$, the output indicator OUT-09 is the apnea index mean $y_2$, and a conversion mathematical relationship corresponding to the apnea index mean is:

$$y_2 = f_2(x_1, x_2, x_3)$$

where, a parameter of the function $f_2$ is determined based on the original mapping matrix and element values of a column (i.e., column 1) where the apnea index mean is located in at least one target mapping matrix (the 1st to 8th target mapping matrices are taken as an example here). For example, a relationship between the apnea index mean and the hypopnea index, the apnea index and the pressure parameter is a weighted sum, and the corresponding conversion mathematical relationship may be specifically:

$$y_2 = w_1 * x_1 + w_2 * x_2 + w_3 * x_3$$

where, $w_1$, $w_2$ and $w_3$ are determined based on the original mapping matrix and element values of a column where the apnea index mean is located in the at least one target mapping matrix. For example, in the above Table 12, element values in a first column of a row where the input indicator IN-01 is located are respectively: 0.50000006, 0.500000056, 5.55500801E-08, 5.55500801E-08, 5.55500801E-08, 5.55500801E-08, 5.55500801E-08, 5.55500801E-08. Then, the value range of w1 may be [5.55500801E-08, 0.50000006]. Similarly, it may be determined that the value range ofwz is [0.50000006, 1.00000006], and the value range of w3 is [2.22044605E-17, 2.22044605E-15].

As verified by the inventor, in order to achieve a more accurate calculation result, in the conversion mathematical relationship corresponding to the apnea index mean, the value of $w_1$ may be specifically 0.50000006, 0.500000056 or 5.55500801E-08, the value of $w_2$ may be specifically 0.50000006 or 1.00000006, and the value of $w_3$ may be specifically 2.22044605E-16.

For yet another example, the input indicator IN-01 is the hypopnea index $x_1$, the input indicator IN-06 is the apnea index $x_2$, and the input indicator IN-07 is the pressure parameter $x_3$, the output indicator OUT-10 is the hypopnea index mean $y_3$, and a conversion mathematical relationship corresponding to the hypopnea index mean is:

$$y_3 = f_3(x_1, x_2, x_3)$$

where, a parameter of the function $f_3$ is determined based on the original mapping matrix and element values of a column (i.e., column 1) where the hypopnea index mean is located in at least one target mapping matrix (the 1st to 8th target mapping matrices are taken as an example here). For example, a relationship between the hypopnea index mean and the hypopnea index, the apnea index and the pressure parameter is a weighted sum, and the corresponding conversion mathematical relationship may be specifically:

$$y_3 = w_1 * x_1 + w_2 * x_2 + w_3 * x_3$$

where, w1, w2 and w3 are determined based on the original mapping matrix and element values of a column where the hypopnea index mean is located in the at least one target mapping matrix. For example, in the above Table 12, element values in a first column of a row where the input indicator IN-01 is located are respectively: 0.50000006, 0.500000056, 1.00000006, 1.00000006, 1.00000006, 1.00000006, 1.00000006, 1.00000006. Then, the value range of w1 may be [0.500000056, 1.00000006]. Similarly, it may be determined that the value range of w2 is [5.55500802E-08, 0.50000006], and the value range of w3 is [-7.85046229E-16, -7.85046229E-18].

As verified by the inventor, in order to achieve a more accurate calculation result, in the conversion mathematical relationship corresponding to the hypopnea index mean, the value of $w_1$ may be specifically 0.50000006, 0.500000056 or 1.00000006, the value of $w_2$ may be specifically 5.55500802E-08 or 0.50000006, and the value of $w_3$ may be specifically -7.85046229E-17.

That is, the data processing module is configured to train and obtain value ranges of relational parameters w1, w2 and w3 in the following manner.

Sample medical data and sample business data are obtained. The sample medical data includes 9 input indicators, and the sample business data includes 10 output indicators. The input indicators include the hypopnea index x1, the apnea index x2 and the pressure parameter x3, the output indicators include the sleep apnea hypopnea index mean y1, the apnea index mean y2, and the hypopnea index mean y3.

An original mapping matrix R is constructed based on the obtained sample medical data and sample business data. An element R (i, j) in the original mapping matrix is used to represent an relationship between the input indicator i in the sample medical data and the output indicator j in the sample business data, i and j are positive integers, and i ∈ [1, 9], j∈[1, 10]. When there is a correlation between the input indicator i and the output indicator j, R (i, j)=1, and when it cannot be determined that whether there is the correlation between the input indicator i and the output indicator j, R (i, j)=0.

A first matrix P and a second matrix Q are obtained based on the original mapping matrix R. The first matrix represents a degree of influence of the input indicator on each output indicator, and the second matrix represents a degree of the output indicator being influenced by each input indicator.

The step of obtaining the first matrix P and the second matrix Q based on the original mapping matrix R includes:

performing singular value decomposition on the original mapping matrix R to obtain a singular value Σ[1.73205081,1,1,1,1,1,1,1,1,1] of the original mapping matrix R, the first matrix P, and the second matrix Q, where the first matrix P is a 9*9-dimensional matrix, and the second matrix Q is a 10*10-dimensional matrix.

taking the first k element values of the singular value Σ as matrix diagonal element values to construct a plurality of intermediate matrices S, where k is a positive integer, and k E [1, 8], and the plurality of intermediate matrices S are all 9*10-dimensional matrices;

determining a plurality of target mapping matrices Rk according to a product of the first matrix P, the second matrix Q and each intermediate matrix S; and

determining value ranges of the relational parameters w1, w2 and w3 based on the original mapping matrix R and element values of a column where the output indicator is located in the plurality of target mapping matrices Rk.

The step of determining the value ranges of the relational parameters w1, w2 and w3 based on the original mapping matrix R and the element values of the column where the output indicator is located in the plurality of target mapping matrices Rk includes:

summarizing element values of columns where the output indicator j is located in the plurality of target mapping matrices Rk to obtain elements of a column where the output indicator j is located when values of k of the intermediate matrix S are different; and

determining the value ranges of the relational parameters w1, w2 and w3 based on the elements of the column where the output indicator j is located when values of k of the intermediate matrix S are different.

The above data conversion and model training processes may be completed by the data processing module of the data lake cluster.

The method for determining the parameter of the function *f* is exemplified in the above embodiments. Those skilled in the art can easily understand that in other embodiments of the present disclosure, depending on a main algorithm of the function *f*, its parameter may also be determined in other ways using the data in the above Table 12, which also falls within the scope of the present disclosure.

Referring to FIG. 11, in an embodiment of the present disclosure, the emergency treatment system may further include a dispatch center terminal 1101. In addition, it may further include one or more of an alarm terminal 1102, a rescuer terminal 1103, and a driver terminal 1104.

The alarm terminal 1102 can be communicatively connected with the dispatch center terminal 1101 and is configured to send alarm information to the dispatch center terminal 1101. In an embodiment of the present disclosure, the alarm terminal 1102 is a device capable of collecting the user's voice, image, video, position and other information. For example, the alarm terminal 1102 may be a mobile electronic device, such as a smartphone, a smart watch, or a smart bracelet. The alarm terminal 1102 may also be an Internet of Things device in a community, park, or factory, such as a monitoring device or other information collection device. After the user triggers an alarm function of the alarm terminal 1102, the alarm terminal 1102 can establish the communication connection with the dispatch center terminal 1101, so as to collect the user's voice, image, video, position or other information and transmit it to the dispatch center terminal 1101.

The dispatch center terminal 1101 is configured to receive the alarm information and obtain a position of the subject to be treated, and dispatch, according to a preset dispatch algorithm, an ambulance vehicle (for example, and an ambulance resource) to the position of the subject to be treated to provide treatment. In an embodiment of the present disclosure, the dispatch center terminal 1101 may be deployed with a distributed agent system and a comprehensive display system (such as a large LCD splicing screen), so as to facilitate dispatching and outing of the ambulance vehicle, the deployment of the emergency medical personnel in the hospital, patient condition and treatment arrangements, etc. through the preset dispatch algorithm combined with manual coordination and management. In embodiments of the present disclosure, the preset dispatch algorithm may include, for example, establishing a regional road network model, and generating an optimal path according to an emergency plan and the regional road network model, and then dispatching the ambulance vehicle or the like based on the regional road network model and the optimal path.

In embodiments of the present disclosure, the dispatch center terminal 1101 may further be configured to: intelligently identify an emergency scene based on the received each piece of alarm information; when receiving new alarm information, determine whether to configure a new ambulance vehicle and a new ambulance resource based on the ambulance vehicle and the ambulance resource configured for the identified emergency scene if it is determined that the new alarm information is located in the identified emergency scene.

For example, the dispatch center terminal 1101 may intelligently identify the emergency scene through audio and video semantic analysis, big data processing and other technologies based on the alarm information generated in similar positions (such as within 1 kilometer, etc.) within a certain period of time (such as within 1 hour, etc.), and record it. When the new alarm information is received, it may be determined whether the new alarm information is located in the identified emergency scene according to a scene corresponding to the new alarm information. If it is determined that the new alarm information is located in the identified emergency scene, and the ambulance vehicle and the ambulance resource configured for the identified emergency scene can already meet a treatment plan corresponding to the new alarm information, there is no need to configure the new ambulance vehicle and ambulance resource. Otherwise, an additional ambulance vehicle and an additional ambulance resource will be dispatched to meet the treatment plan corresponding to the new alarm information.

In addition, the dispatch center terminal 1101 may also synchronize the alarm information to an emergency station in the community or factory area near an alarm caller or other institutions that can provide first aid services based on a position of the alarm caller and other information, thereby facilitating an on-duty volunteer or other rescuers to launch rescue operations as soon as possible, fully utilizing first aid resources, and avoiding delays in precious golden first aid time.

The rescuer terminal 1103 is communicatively connected with the dispatch center terminal 1101 and the medical business system, and is configured to receive dispatch information from the dispatch center terminal 1101. For example, the rescuer terminal 1103 may also receive medical data related to a treatment subject. As an example, medical record information of the treatment subject may be entered through the rescuer terminal 1103. In embodiments of the present disclosure, the rescuer terminal 1103 may be, for example, a dedicated communication terminal, or a smartphone installed with a relevant application, which can facilitate the rescuer to receive the dispatch information of the dispatch center terminal 1101, and also facilitate the rescuer to feed back information to the dispatch center terminal 1101.

In embodiments of the present disclosure, the dispatch center terminal 1101 may further be configured to dispatch an internal rescuer of the medical institution to provide remote assistance based on the medical data related to the subject to be treated. Specifically, as mentioned above, the medical institution may obtain the real-time medical data and the historical medical data of the subject to be treated from the data lake cluster by means of the medical business system in embodiments of the present disclosure. Therefore, the internal rescuer of the medical institution can use this to provide the remote assistance, such as a remote diagnosis, a remote device operation, etc. In addition, it can also facilitate the medical institution to carry out relevant preparations in advance, further reducing the waste of treatment time.

In addition, in the conventional emergency dispatch system, a treatment capacity and bed information of the medical institution cannot be known in time in the emergency vehicle dispatching and transportation process, which may cause a situation where it is found that the medical institution does not have the treatment capacity upon arriving at the medical institution, resulting in multiple transfers, which wastes the first aid resource and delays the precious golden first aid time. In embodiments of the present disclosure, in order to cope with the rapid real-time changes in the corresponding ambulance resources during the treatment and transportation process and avoid the occurrence of multiple transfers, the dispatch center terminal 1101 may further obtain first aid information (e.g., abed condition, a treatment resource) of each hospital in real time, thereby facilitating the dispatch center terminal 1101 to guide the ambulance vehicle to transport the subject to be treated to the preferred medical institution; at the same time, the dispatch center terminal 1101 may also send the treatment capacity and the bed information of the medical institution to the rescuer terminal 1103, so that the rescuer can more accurately determine whether the transfer is needed, thereby reducing the impact of sudden changes in the bed condition.

The driver terminal 1104 is communicatively connected with the dispatch center terminal 1101 and is configured to guide a driver to drive the ambulance vehicle to the position of the subject to be treated. The driver terminal 1104 may be a vehicle-mounted terminal, such as a vehicle-mounted central control screen, a vehicle-mounted navigator, etc. Alternatively, the driver terminal 1104 may also be a smartphone or other communication terminal installed with a relevant application.

In some implementations, the driver terminal 1104 may display a current traveling process in real time. For example, when the ambulance vehicle arrives at the alarm position, a display state is "arrived at the alarm position"; when the ambulance vehicle is rushing to the treatment hospital, the display state is "on the way"; and when the ambulance vehicle arrives at the treatment hospital, the display state is "arrived at the hospital", and the like.

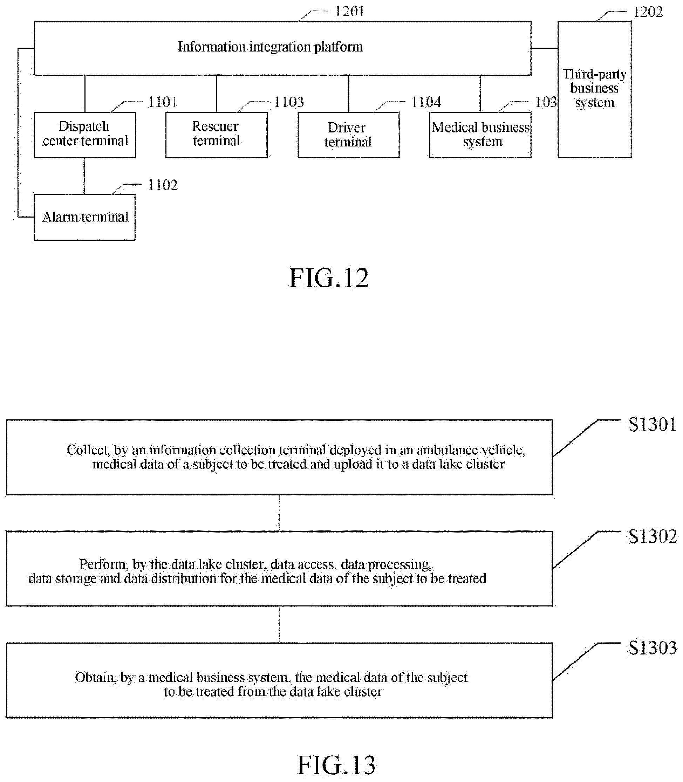

Referring to FIG. 12, in an embodiment of the present disclosure, the emergency treatment system may further include an information integration platform 1201, which is in communication with at least one of the management terminal 701, the dispatch center terminal 1101, the alarm terminal 1102, the rescuer terminal 1103, the driver terminal 1104 and the medical business system 103.

The information integration platform 1201 is configured to retrieve a personal health file and/or a knowledge base from a third-party business system 1202, and send them to the at least one of the management terminal 701, the dispatch center terminal 1101, the alarm terminal 1102, the rescuer terminal 1103, the driver terminal 1104 and the medical business system 103; and/or,

the information integration platform 1201 is configured to obtain information of the subject to be treated from the at least one of the management terminal 701, the dispatch center terminal 1101, the alarm terminal 1102, the rescuer terminal 1103, the driver terminal 1104 and the medical business system 103.

In some embodiments, the personal health file, such as historical medical information, medical history, an inspection report, etc., in the third-party business system 1202 is integrated through the information integration platform 1201, and sent to the at least one of the management terminal 701, the dispatch center terminal 1101, the alarm terminal 1102, the rescuer terminal 1103, the driver terminal 1104 and the medical business system 103. The user information (including the medical record information) stored in these terminals is integrated and compared with the personal health file in the information integration platform 1201 to facilitate a more comprehensive understanding of the patient's personal health condition. For example, key information such as a mental illness, an infectious disease, an examination record, etc. may also be extracted and displayed to the dispatch center terminal 1101, which facilitates a dispatcher to quickly make a prediction of the alarm situation.

In some implementations, the information integration platform 1201 is integrated with the knowledge base in the third-party business system 1202, and the knowledge base may be synchronized to the dispatch center terminal 1101, the rescuer terminal 1103, etc., to facilitate the standardized processing.

In some implementations, the dispatch center terminal 1101 can synchronize the dispatch information of the dispatcher during the dispatch process, such as a preliminary diagnosis, graphic guidance, an audio and video, etc., to the information integration platform 1201, and synchronize the treatment information to the information integration platform 1201 through the rescuer terminal 1103, etc.

In some implementations, the information integration platform 1201 may include a decision evaluation module, and the decision evaluation module evaluates the information of the subject to be treated based on the knowledge base. An effective evaluation is performed uniformly by the decision evaluation module. Based on an evaluation result, the relevant personnel can make targeted enhancements and update the knowledge base at the same time. For example, the decision evaluation module predicts a condition of the subject to be treated based on the knowledge base, or provides a standard treatment method based on the knowledge base.

In some embodiments, the information integration platform 1201 may further be configured to: intelligently identify an emergency scene based on the received each piece of alarm information; when receiving new alarm information, determine whether to configure a new ambulance vehicle and a new ambulance resource based on the ambulance vehicle and the ambulance resource configured for the identified emergency scene if it is determined that the new alarm information is located in the identified emergency scene.

It can be understood that at least some of the above functions may also be implemented by the dispatch center terminal 1101. For example, after the information integration platform 1201 completes the intelligent identification of the emergency scene, it sends scene information to the dispatch center terminal 1101, and the dispatch center terminal 1101 dispatches the ambulance vehicle and the ambulance resource based on specific conditions.

For example, the emergency scene is identified as a collective public event based on the received each piece of alarm information, and there may be a situation where a plurality of persons give alarms at a position of the collective public event. For example, the emergency scene may be identified based on the information provided by the person who give the alarm, or through environmental information such as voice and video. In the case that the plurality of persons give the alarms in the same area, the information integration platform 1201 can assign a certain number of ambulance vehicles and configure corresponding ambulance resources based on an analysis result. For example, when two people with minor injuries give alarms, it is considered that the assignment of one ambulance vehicle is enough, so as to save the ambulance resource.

For example, the dispatch center terminal 1101 may intelligently identify the emergency scene through audio and video semantic analysis, big data processing and other technologies based on the alarm information generated in similar positions (such as within 1 kilometer, etc.) within a certain period of time (such as within 1 hour, etc.), and record it. When the new alarm information is received, it may be determined whether the new alarm information is located in the identified emergency scene according to a scene corresponding to the new alarm information. If it is determined that the new alarm information is located in the identified emergency scene, and the ambulance vehicle and the ambulance resource configured for the identified emergency scene can already meet a treatment plan corresponding to the new alarm information, there is no need to configure the new ambulance vehicle and ambulance resource. Otherwise, an additional ambulance vehicle and an additional ambulance resource will be dispatched to meet the treatment plan corresponding to the new alarm information.

For example, the alarm terminal 1102, when making an alarm, uploads alarm information (the position, the audio and video), etc. to the information integration platform 1201. The dispatch center terminal 1101 uploads the dispatch information (the preliminary diagnosis, the vehicle assignment, the operation record, the audio and video and other information) to the emergency information integration platform 1201. The dispatcher may mark an emergency level (such as very urgent, normal urgent, not urgent, etc.) at the dispatch center terminal 1101, and the emergency level is summarized to the dispatch information and uploaded to the information integration platform 1201. The information integration platform 1201 intelligently identifies the emergency scene based on the number of alarms generated in similar positions during the latest time, the audio and video semantic analysis, etc., synchronizes the emergency condition to the dispatch center terminal 1101, and at the same time, after a new alarm occurs, may intelligently determines, based on the newly summarized alarm information, whether the described scene and symptom are the same and whether the vehicle has been assigned. and conduct a targeted prompt. If the information integration platform 1201 identifies the emergency scene, it can summarize the existing alarm information and the dispatch information and display them in the 120 emergency command platform according to a geographic location in the dispatch information and a scene description in the audio and video semantic analysis, which facilitates timely emergency command and unified coordination.

In addition, the dispatch center terminal 1101 and/or the information integration platform 1201 may also synchronize the alarm information to an emergency station in the community or factory area near an alarm caller or other institutions that can provide first aid services based on a position of the alarm caller and other information, thereby facilitating an on-duty volunteer or other rescuers to launch rescue operations as soon as possible, fully utilizing first aid resources, and avoiding delays in precious golden first aid time.

In some embodiments, the rescuer terminal 1103 is further configured to record the treatment information, and the information integration platform 1201 is configured to draw the treatment information records as a timeline according to a chronological order and send it to the medical business system 103.

For example, the rescuer terminal 1103 collects an on-site video and identifies patient identity information, and uploads them to the information integration platform 1201 in a unified manner. A doctor can, by means of the rescuer terminal 1103, take real-time photos or videos of or manually input the physical, drug and other treatment operations carried out, for the upload to the information integration platform 1201 in real time, and the operation time is recorded upon uploading. The information integration platform 1201 forms a timeline of in-vehicle operations and summarize with location information for displaying to the medical information system. The information integration platform 1201 intelligently sorts patients who are about to arrive at the hospital based on treatment information, condition, distance, etc., so that emergency doctors determines a time to arrive at the hospital based on a time of treatment in the vehicle and a position of the vehicle, customizes a rescue plan, and coordinates hospital resources.

In some embodiments, the information integration platform 1201 is configured to receive hospital resource information sent from the medical business system 103 and send it to the dispatch center terminal 1101.

For example, through the information integration platform 1201, each hospital can, through the medical information system, timely update and synchronize the bed condition, the treatment capability, etc. of each hospital to the information integration platform 1201. The dispatcher can perform the selective dispatch according to an empty bed condition at the dispatch center terminal 1101. During the process of transporting the patient, the doctor in the vehicle can check a real-time bed condition of the heading-to hospital at the rescuer terminal 1103, which facilitates timely confirmation of the need to transfer to a hospital and reduces the impact caused by a sudden bed change.

It should be noted that although several modules or units of devices for executing actions are mentioned in the above detailed description, such division of modules or units is not mandatory. In fact, features and functions of two or more of the modules or units described above may be embodied in one module or unit in accordance with embodiments of the present disclosure. Alternatively, the features and functions of one module or unit described above may be further divided into multiple modules or units.

Various component embodiments of the present disclosure may be implemented in hardware, or in software modules running on one or more processors, or in a combination thereof.

Furthermore, there is further provided an emergency treatment method in embodiments of the present disclosure. The emergency treatment method may be applied to an emergency treatment system. Referring to FIG. 13, the emergency treatment method may include steps S1301 to S1303.

In the step S1301, medical data of a subject to be treated is collected by an information collection terminal deployed in an ambulance vehicle, and uploaded to a data lake cluster.

In the step S1302, data access, data processing, data storage and data distribution are performed for the medical data of the subject to be treated, by the data lake cluster.

In the step S1303, the medical data of the subject to be treated is obtained from the data lake cluster by a medical business system.

Specific details of each step in the above emergency treatment method have been described in detail in the corresponding emergency treatment system, which will not be described again here.

It should be understood that although the steps in the flowchart of the drawings are shown in an order as indicated by the arrows, those steps are not necessarily performed in the order as indicated by the arrows. The steps are not strictly limited to their current order, but may be performed in other orders, unless explicitly stated herein. Furthermore, at least a portion of the steps in the flowcharts of the drawings may include a plurality of sub-steps or stages, which are not necessarily performed at the same time, but may be performed at different times, and the order of execution thereof is not necessarily performed sequentially, and may be performed in turn or alternately with other steps or at least a portion of the sub-steps or stages of other steps.

In an embodiment of the present disclosure, there is further provided an electronic device, including: a processor; a memory configured to store instructions executable by the processor; and the processor is configured to execute the method described in any one of embodiments of the present disclosure.

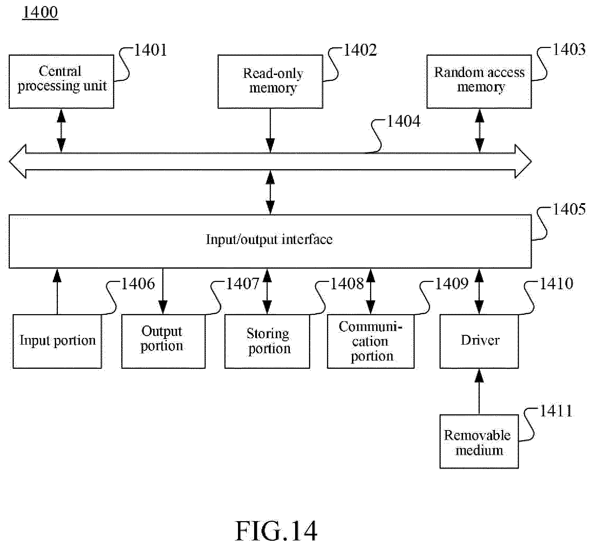

FIG. 14 shows a schematic structural diagram of a computer system configured to implement an electronic device according to an embodiment of the present disclosure. It should be noted that the computer system 1400 of the electronic device shown in FIG. 14 is only an example, and should not impose any restrictions on functions and scope of use of embodiments of the present disclosure.

As shown in FIG. 14, the computer system 1400 includes a central processing unit 1401 that may perform various appropriate actions and processes based on a program stored in a read-only memory 1402 or a program loaded into a random access memory 1403 from a storing portion 1408. The random access memory 1403 further stores various programs and data required for the system operation. The central processing unit 1401, the read-only memory 1402 and the random access memory 1403 are connected to each other via a bus 1404. An input/output interface 1405 is also connected to the bus 1404.

The input/output interface 1405 is connected with the following components: an input portion 1406 including a keyboard, a mouse, etc.; an output portion 1407 including such as a cathode ray tube (CRT), a liquid crystal display (LCD), etc. and a speaker, etc.; a storing portion 1408 including a hard disk, etc.; and a communication portion 1409 including a network interface card such as a local area network (LAN) card, a modem, etc. The communication portion 1409 performs communication processing via a network such as the Internet. A driver 1410 is also connected to the input/output interface 1405 as needed. A removable medium 1411, such as a disk, an optical disk, a magnetic disk, a semiconductor memory, etc., is mounted on the driver 1410 as needed, such that the computer programs as read from the removable medium 1411 may be mounted into the storing portion 1408 as needed.

In particular, according embodiments of the present disclosure, the process described above with reference to the flowchart may be implemented as a computer software program. For example, embodiments of the present disclosure include a computer program product including a computer program carried on a computer-readable medium, and the computer program includes a program code for performing the method shown in the flowchart. In such an embodiment, the computer program may be downloaded and installed from the network via the communication portion 1409, and/or from the removable medium 1411. The computer program, when executed by the central processing unit 1401, performs various functions as defined in the device of the present disclosure.

In embodiments of the present disclosure, there is further provided a non-volatile computer-readable storage medium having a computer program stored thereon, which, when executed by a computer, causes the computer to execute any of the above methods.

It should be noted that the non-volatile computer-readable storage medium may be, for example, but not limited to, an electrical, magnetic, optical, electromagnetic, infrared, or semiconductor system, apparatus or device, or any combination thereof. Examples of the computer-readable storage medium may include, but are not limited to: an electrical connection with at least one wire, a portable computer disk, a hard disk, a random-access memory, a read-only memory, an erasable programmable read-only memory (EPROM or flash memory), an optical fiber, a portable compact disk read-only memory (CD-ROM), an optical storing device, a magnetic storing device, or any suitable combination thereof. In the present disclosure, the computer-readable storage medium may be any tangible medium that includes or stores a program, and the program may be adopted by or in combination with an instruction executing system, apparatus or device. In addition, in the present disclosure, the computer-readable signal medium may include a data signal propagated in a baseband or as part of a carrier wave that carries a computer-readable program code. The data signals as propagated may take a variety of forms, including but not limited to electromagnetic signals, optical signals, or any suitable combination thereof. The computer-readable signal medium may also be any computer-readable medium that sends, propagates, or transmits the program as adopted by or in combination with the instruction executing system, apparatus or device. The program code included on the computer-readable medium may be transmitted via any suitable medium, including but not limited to wireless, wire, fiber optic cable, radio frequency, etc., or any suitable combination thereof.

Other embodiments of the present disclosure will be apparent to those skilled in the art from consideration of the specification and practice of the present disclosure disclosed herein. The present application is intended to cover any variations, uses, or adaptations of the present disclosure, which are in accordance with the general principles of the present disclosure and include common general knowledge or conventional technical means in the art that are not disclosed in the present disclosure. The specification and embodiments are illustrative, and the real scope and spirit of the present disclosure is defined by the appended claims.

**Claims**

1. An emergency treatment system, comprising:

   an information collection terminal, deployed in an ambulance vehicle, and configured to collect medical data of a subject to be treated and upload the medical data to a data lake cluster;
   the data lake cluster, configured to perform data access, data processing, data storage and data distribution for the medical data of the subject to be treated; and
   a medical business system, configured to obtain the medical data of the subject to be treated from the data lake cluster.

2. The emergency treatment system according to claim 1, wherein the data lake cluster comprises:

   a data access module, configured to determine a corresponding target access manner according to a type of the information collection terminal and access the medical data of the subject to be treated in the target access manner;
   a data processing module, configured to analyze indicator information of the medical data of the subject to be treated, and establish a mapping relationship between each piece of indicator information and a standard indicator; and

a data distribution module, configured to distribute to the medical business system the medical data of the subject to be treated processed by the data processing module.

3. The emergency treatment system according to claim 2, wherein analyzing in the data processing module the indicator information of the medical data of the subject to be treated comprises:

analyzing, based on a data analysis model, the indicator information of the medical data of the subject to be treated uploaded by the information collection terminal; and
wherein the data analysis model is obtained by modeling based on an analytical protocol corresponding to the information collection terminal.

4. The emergency treatment system according to claim 3, wherein the indicator information of the medical data of the subject to be treated comprises n fields, wherein an i-th field represents a (i-1)*m+1-th bit to an i*m-th bit of a binary value of an indicator of the medical data of the subject to be treated; and the indicator information of the medical data of the subject to be treated is analyzed by the data analysis model through the following formula:

$$y = \sum_{i=1}^{n} f_2\left[f_1(x), i\right] * (2^m)^{i-1}$$

where the function $f_1(x)$ is configured to convert an object into a binary value, and $f_2(x)$ is configured to convert an object into a decimal value.

5. The emergency treatment system according to claim 2, wherein the standard indicator at least comprises a standard code identity, and establishing the mapping relationship between each piece of indicator information and the standard indicator in the data processing module comprises:

after analyzing the indicator information of the medical data of the subject to be treated uploaded by the information collection terminal, obtaining a non-standard code identity therein;
based on a mapping relationship between the non-standard code identity and the standard code identity, performing key-value storage for the standard code identity and an indicator value in the indicator information of the medical data of the subject to be treated; and
associating the standard code identity corresponding to the medical data of the subject to be treated with a device identity of the information collection terminal.

6. The emergency treatment system according to claim 2, wherein:

a first message platform is configured between the data access module and the data processing module, and the first message platform is used by the data access module to transmit accessed information to the data processing module in a message service manner; and
a second message platform is configured between the data distribution module and the medical business system, and the second message platform is used by the data distribution module to transmit the processed medical data of the subject to be treated to the medical business system in the message service manner.

7. The emergency treatment system according to claim 6, wherein the data distribution module is further configured to send the medical data of the subject to be treated to a message queue for the second message platform to pull the medical data of the subject to be treated from the message queue.

8. The emergency treatment system according to claim 2, wherein a data conversion relationship exists between an input indicator in the medical data of the subject to be treated accessed by the data access module and an output indicator in the processed medical data of the subject to be treated distributed by the data distribution module.

9. The emergency treatment system according to claim 8, wherein:

the input indicator comprises a hypopnea index x1, an apnea index x2 and a pressure parameter x3;
the output indicator comprises a sleep apnea hypopnea index mean y1; and

the following data conversion relationship exists between y1 and x1, x2 and x3:
y1=f1(x1,x2,x3), where f1 represents a linear conversion relationship or a nonlinear conversion relationship.

10. The emergency treatment system according to claim 9, wherein the data conversion relationship is:
y1=w1*x1+w2*x2+w3*x3, where w1, w2 and w3 are relational parameters.

11. The emergency treatment system according to claim 10, wherein a value range of w1 is [1,1.00000011], a value range of w2 is [1,1.00000011], and a value range of w3 is [7.85046229E -18,7.85046229E-16].

12. The emergency treatment system according to claim 11, wherein a value of w1 is 1.00000011, a value of w2 is 1.00000011, and a value of w3 is 7.85046229E-17.

13. The emergency treatment system according to claim 8, wherein:

the input indicator comprises a hypopnea index x1, an apnea index x2 and a pressure parameter x3;
the output indicator is an apnea index mean y2; and
the following data conversion relationship exists between y2 and x1, x2 and x3:
y2=f2(x1,x2,x3), where f2 represents a linear conversion relationship or a nonlinear conversion relationship.

14. The emergency treatment system according to claim 13, wherein the data conversion relationship is:
y2=w1*x1+w2*x2+w3*x3, where w1, w2 and w3 are relational parameters.

15. The emergency treatment system according to claim 14, wherein a value range of w1 is [5.55500801E-08, 0.50000006], a value range of w2 is [0.50000006, 1.00000006], and a value range of w3 is [2.22044605E-17, 2.22044605E-15].

16. The emergency treatment system according to claim 15, wherein a value of w1 is 0.50000006, 0.500000056 or 5.55500801E-08, a value of w2 is 0.50000006 or 1.00000006, and a value of w3 is 2.22044605E-16.

17. The emergency treatment system according to claim 8, wherein:

the input indicator comprises a hypopnea index x1, an apnea index x2 and a pressure parameter x3;
the output indicator is a hypopnea index mean y3; and
the following data conversion relationship exists between y3 and x1, x2 and x3:
y3=f3(x1,x2,x3), where f3 represents a linear conversion relationship or a nonlinear conversion relationship.

18. The emergency treatment system according to claim 17, wherein the data conversion relationship is:
y3=w1*x1+w2*x2+w3*x3, where, w1, w2 and w3 are relational parameters.

19. The emergency treatment system according to claim 18, wherein a value range of w1 is [0.500000056, 1.00000006], a value range of w2 is [5.55500802E-08, 0.50000006], and a value range of w3 is [-7.85046229E-16, -7.85046229E-18].

20. The emergency treatment system according to claim 19, wherein a value of w1 is 0.50000006, 0.500000056 or 1.00000006, a value of w2 is 5.55500802E-08 or 0.50000006, and a value of w3 is -7.85046229E-17.

21. The emergency treatment system according to claim 8, wherein the data conversion relationship between the medical data of the subject to be treated accessed by the data access module and the processed medical data of the subject to be treated distributed by the data distribution module is obtained in the following manner:

obtaining an original mapping matrix R between sample medical data and sample business data, wherein the original mapping matrix R is an M×N matrix, and an element R(i,j) is used to represent a relationship between an input indicator i in the sample medical data and an output indicator j in the sample business data;
for a number of dimensions k, obtaining a first matrix P of M×k and a second matrix Q of k×N with P×Q=R as a target, and calculating a target mapping matrix Rk based on the first matrix P and the second matrix Q; and
determining a data conversion relationship between each input indicator and an output indicator in business data according to the original mapping matrix and at least one target mapping matrix;
where m and n are integers greater than 1, i, j, and k are positive integers, and $i \in [1,m]$, $j \in [1,n]$, and $k \in [1,m-1]$.

**22.** The emergency treatment system according to claim 21, wherein the data conversion relationship between each input indicator and the output indicator in the business data is determined according to the original mapping matrix and 1st to (n-1)-th target mapping matrices.

**23.** The emergency treatment system according to claim 21, wherein the data conversion relationship is:
$y_j=f(x_i, i \in [1,m])$, where $y_j$ is a value of the output indicator j, a parameter of the function f is determined based on the original mapping matrix and an element value of a column where the output indicator j is located in the at least one target mapping matrix, and $x_i$ is a value of the input indicator i.

**24.** The emergency treatment system according to claim 21, wherein:

the input indicator comprises a hypopnea index x1, an apnea index x2 and a pressure parameter x3;
the output indicator is a sleep apnea hypopnea index mean y1; and
a conversion relationship corresponding to the sleep apnea hypopnea index mean is:
$y1=f1(x1,x2,x3)$, where a parameter of the function f1 is determined based on the original mapping matrix and an element value of a column where the sleep apnea hypopnea index mean is located in the at least one target mapping matrix.

**25.** The emergency treatment system according to claim 24, wherein the conversion relationship corresponding to the sleep apnea hypopnea index mean is specifically:
$y1=w1*x1+w2*x2+w3*x3$, wherein w1, w2 and w3 are determined based on the original mapping matrix and the element value of the column where the sleep apnea hypopnea index is located in the at least one target mapping matrix.

**26.** The emergency treatment system according to claim 21, wherein:

the input indicator comprises a hypopnea index x1, an apnea index x2 and a pressure parameter x3;
the output indicator is an apnea index mean y2; and
a conversion relationship corresponding to the apnea index mean is:
$y2=f2(x1,x2,x3)$, wherein a parameter of the function f2 is determined based on the original mapping matrix and an element value of a column where the apnea index mean is located in the at least one target mapping matrix.

**27.** The emergency treatment system according to claim 26, wherein the conversion relationship corresponding to the apnea index mean is specifically:
$y2=w1*x1+w2*x2+w3*x3$, wherein w1, w2 and w3 are determined based on the original mapping matrix and the element value of the column where the apnea index mean is located in the at least one target mapping matrix.

**28.** The emergency treatment system according to claim 21, wherein:

the input indicator comprises a hypopnea index x1, an apnea index x2 and a pressure parameter x3;
the output indicator is a hypopnea index mean y3; and
a conversion relationship corresponding to the hypopnea index mean is:
$y3=f3(x1,x2,x3)$, wherein a parameter of the function f3 is determined based on the original mapping matrix and an element value of a column where the hypopnea index mean is located in the at least one target mapping matrix.

**29.** The emergency treatment system according to claim 28, wherein the conversion relationship corresponding to the hypopnea index mean is specifically:
$y3=w1*x1+w2*x2+w3*x3$, wherein w1, w2 and w3 are determined based on the original mapping matrix and the element value of the column where the hypopnea index mean is located in the at least one target mapping matrix.

**30.** The emergency treatment system according to claim 1, further comprising a management terminal;
the management terminal is configured to:

record a device start of use time and a device identity of the information collection terminal;
receive the medical data distributed from the data lake cluster; and
associate the medical data with user information of the subject to be treated according to the device identity and the device start of use time;
the medical business system is further configured to receive the medical data associated with the user information of the subject to be treated.

**31.** The emergency treatment system according to claim 30, wherein the management terminal is further configured to store historical medical data of the subject to be treated, and jointly distribute the historical medical data and real-time medical data of the subject to be treated to the medical business system.

**32.** The emergency treatment system according to claim 30, wherein the management terminal is further configured to store medical data of relatives and friends of the subject to be treated, and jointly distribute the medical data of the relatives and friends of the subject to be treated and the medical data of the subject to be treated to the medical business system.

**33.** The emergency treatment system according to claim 30, wherein the management terminal is further configured to receive a query request, and obtain, according to a query condition comprised in the query request, relevant data from the data lake cluster to generate a query result.

**34.** The emergency treatment system according to claim 30, wherein the management terminal further comprises:
a data visualization module, configured to generate visual content according to data of the data lake cluster to display the visual content through a front-end page.

**35.** The emergency treatment system according to claim 30, wherein the management terminal further comprises:
a device parameter monitoring module, configured to record and analyze behavior information of the information collection terminal to determine whether the information collection terminal is an abnormal terminal.

**36.** The emergency treatment system according to any one of claims 31 to 35, wherein the emergency treatment system further comprises:
a dispatch center terminal, configured to receive alarm information and obtain a position of the subject to be treated, and dispatch, according to a preset dispatch algorithm, the ambulance vehicle to the position of the subject to be treated for treatment.

**37.** The emergency treatment system according to claim 36, wherein the dispatch center terminal is further configured to dispatch, according to the medical data related to the subject to be treated, an internal rescuer of a medical institution to provide remote assistance.

**38.** The emergency treatment system according to claim 36, wherein the emergency treatment system further comprises:
an alarm terminal, available for being in a communication connection with the dispatch center terminal, and configured to send alarm information to the dispatch center terminal.

**39.** The emergency treatment system according to claim 36, wherein the emergency treatment system further comprises:
a rescuer terminal, communicatively connected with the dispatch center terminal, and configured to receive dispatch information of the dispatch center terminal, and enter medical record information of the subject to be treated.

**40.** The emergency treatment system according to claim 36, wherein the emergency treatment system further comprises:
a driver terminal, communicatively connected with the dispatch center terminal and configured to guide a driver to drive the ambulance vehicle to the position of the subject to be treated.

**41.** The emergency treatment system according to any one of claims 30-35 or 37-40, wherein the emergency treatment system further comprises:

an information integration platform in communication with at least one of the management terminal, the dispatch center terminal, the alarm terminal, the rescuer terminal, the driver terminal and the medical business system; the information integration platform is configured to retrieve a personal health file and/or a knowledge base from a third-party business system and send them to the at least one of the management terminal, the dispatch center terminal, the alarm terminal, the rescuer terminal, the driver terminal and the medical business system; and/or,
the information integration platform is configured to obtain information of the subject to be treated from the at least one of the management terminal, the dispatch center terminal, the alarm terminal, the rescuer terminal, the driver terminal and the medical business system.

**42.** The emergency treatment system according to claim 41, wherein the information integration platform comprises a decision evaluation module; and
the decision evaluation module is configured to evaluate the information of the subject to be treated based on the knowledge base.

**43.** The emergency treatment system according to claim 41, wherein the information integration platform is configured to:

identify an emergency scene according to the received information of the subject to be treated; and
upon receiving new alarm information, determining whether to configure a new ambulance vehicle according to an ambulance vehicle configured for the identified emergency scene, if it is determined that the new alarm information is located in the identified emergency scene.

**44.** The emergency treatment system according to claim 41, wherein the rescuer terminal is further configured to record treatment information; and
the information integration platform is configured to draw a treatment information record as a timeline according to a chronological order and send it to the medical business system.

**45.** The emergency treatment system according to claim 41, wherein,
the information integration platform is configured to receive hospital resource information sent from the medical business system and send it to the dispatch center terminal.

**46.** An emergency treatment method, comprising:

collecting, by an information collection terminal deployed in an ambulance vehicle, medical data of a subject to be treated and uploading the medical data to a data lake cluster;
performing, by the data lake cluster, data access, data processing, data storage and data distribution for the medical data of the subject to be treated; and
obtaining, by a medical business system, the medical data of the subject to be treated from the data lake cluster.

**47.** An electronic device, comprising:

a processor; and
a memory, configured to store one or more programs, which when executed by the processor, cause the processor to implement the method according to claim 46.

**48.** A computer-readable storage medium having a computer program stored thereon, which, when executed by a processor, implements the method according to claim 46.

100

101             102             103

# FIG.1

102

Data lake cluster

201

Data access module

202

Data processing module

203

Data storage module

204

Data distribution module

# FIG.2

FIG.3

FE121248012408

| diastolic blood pressure | 120 |
| systolic blood pressure | 90 |
| pulse rate | 88 |
| device identity | XX |

| standard indicator code | non-standard indicator code |
|---|---|
| XY01 | systolic blood pressure |
| XY02 | diastolic blood pressure |
| ML01 | pulse rate |
| ... | ... |
| ... | ... |

| device code | device identity |
|---|---|
| 01 | XX |
| 02 | XX |
| 03 | XX |
| ... | ... |
| ... | ... |

| standard indicator code | indicator value |
|---|---|
| XY01 | 120 |
| XY02 | 90 |
| ML01 | 88 |
| ... | ... |
| ... | ... |

FIG.4

| Configure a standard indicator, the standard indicator at least including a standard code identity | S501 |

| Obtain a non-standard code identity of a non-standard indicator of an information collection terminal, and establish a mapping relationship between the non-standard code identity and a standard code identity | S502 |

| After indicator information of medical data of a subject to be treated uploaded by the information collection terminal is analyzed, obtain the non-standard code identity therein, and perform, based on the mapping relationship between the non-standard code identity and the standard code identity, key-value storage for the standard code identity and an indicator value of the medical data of the subject to be treated | S503 |

| Associate the key-value stored indicator information of the medical data of the subject to be treated with a device identity of the information collection terminal | S504 |

**FIG.5**

Non-standard code identity          Standard code identity

Height ————————————→ H01

Weight

BMI

ShenGao

TiZhong

TZZS ————————————→ B01

W01

**FIG.6**

Data lake cluster

Data access module — 201

Data processing module — 202

Data storage module — 203

Data distribution module — 204

— 102

Management terminal

Device parameter monitoring module — 705

Data query module — 706

— 701

Data visualization module — 707

FIG.7

When a query subject is determined to be a target subject according to a query condition, generate a mark identity for the target subject, and associate the mark identity with a device identity of an information collection terminal corresponding to the target subject — S801

Monitor whether medical data of the target subject is received based on the device identity of the information collection terminal associated with the mark identity — S802

When it is monitored that the medical data of the target subject is received, distribute the medical data of the target subject to a medical institution corresponding to a query request — S803

FIG.8

FIG.9

| | |
|---|---|
| Obtain an original mapping matrix R between sample medical data and sample business data, wherein the original mapping matrix R is an m×n matrix, and an element R(i,j) is used to characterize an relationship between an input indicator i in the sample medical data and an output indicator j in the sample business data | S1001 |
| For the number of dimensions k, obtain a first matrix P of m×k and a second matrix Q of k×n with P×Q=R as a target, and calculate a target mapping matrix Rk based on the first matrix P and the second matrix Q | S1002 |
| Determine a data conversion relationship between each input indicator and an output indicator in the business data according to the original mapping matrix and at least one target mapping matrix | S1003 |

FIG.10

FIG.11

1201

1202

```
┌─────────────────────────────────────────────────────────────┐      ┌──────────────┐
│            Information integration platform                   │      │ Third-party  │
│                                                               │      │ business     │
└─────────────────────────────────────────────────────────────┘      │ system       │
```

1101          1103          1104          103

```
┌──────────┐   ┌──────────┐   ┌──────────┐   ┌──────────────┐
│ Dispatch │   │ Rescuer  │   │ Driver   │   │Medical business│
│center terminal│ terminal │   │ terminal │   │   system     │
└──────────┘   └──────────┘   └──────────┘   └──────────────┘
```

1102

```
┌──────────────┐
│ Alarm terminal│
└──────────────┘
```

## FIG.12

```
┌─────────────────────────────────────────────────────────────┐
│ Collect, by an information collection terminal deployed in an │   S1301
│ ambulance vehicle, medical data of a subject to be treated    │
│ and upload it to a data lake cluster                          │
└─────────────────────────────────────────────────────────────┘

┌─────────────────────────────────────────────────────────────┐
│ Perform, by the data lake cluster, data access, data          │   S1302
│ processing, data storage and data distribution for the        │
│ medical data of the subject to be treated                     │
└─────────────────────────────────────────────────────────────┘

┌─────────────────────────────────────────────────────────────┐
│ Obtain, by a medical business system, the medical data of the │   S1303
│ subject to be treated from the data lake cluster              │
└─────────────────────────────────────────────────────────────┘
```

## FIG.13

1400

FIG.14

## INTERNATIONAL SEARCH REPORT

| International application No. |
|---|
| **PCT/CN2022/103328** |

| A. | CLASSIFICATION OF SUBJECT MATTER |
|---|---|

G16H 40/67(2018.01)i; G16H 50/70(2018.01)i; G16H 80/00(2018.01)i; G16H 10/60(2018.01)i; G06F 9/54(2006.01)i

According to International Patent Classification (IPC) or to both national classification and IPC

| B. | FIELDS SEARCHED |
|---|---|

Minimum documentation searched (classification system followed by classification symbols)

G16H; G06F

Documentation searched other than minimum documentation to the extent that such documents are included in the fields searched

Electronic data base consulted during the international search (name of data base and, where practicable, search terms used)

CNABS; CNTXT; CNKI; SIPOABS; DWPI; USTXT; WOTXT; EPTXT: 急救, 救治, 医疗, 采集, 集群, 数据湖, 分发, 接入, 救护车, 调度, emergency, first aid, medical, collect, group, dataLake, distribute, access, ambulance, schedule

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 113192624 A (BOE TECHNOLOGY GROUP CO., LTD.) 30 July 2021 (2021-07-30) claims 1-48, and description, paragraphs [0074]-[0191] | 1-48 |
| Y | CN 104065743 A (NANJING HEALTH INFORMATION CENTER) 24 September 2014 (2014-09-24) description, paragraphs [0021]-[0053] | 1-48 |
| Y | CN 112463765 A (GUANGZHOU YIBO INFORMATION TECHNOLOGY CO., LTD.) 09 March 2021 (2021-03-09) description, paragraphs [0058]-[0062] | 1-48 |
| A | CN 101977181 A (HANGZHOU MELIORA TECHNOLOGY CO., LTD.) 16 February 2011 (2011-02-16) entire document | 1-48 |
| A | CN 103942454 A (SOUTHEAST UNIVERSITY) 23 July 2014 (2014-07-23) entire document | 1-48 |

☐ Further documents are listed in the continuation of Box C. ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 July 2022** | **29 July 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2022/103328**

| Patent document cited in search report | | | Publication date (day/month/year) | Patent family member(s) | Publication date (day/month/year) |
|---|---|---|---|---|---|
| CN | 113192624 | A | 30 July 2021 | None | |
| CN | 104065743 | A | 24 September 2014 | None | |
| CN | 112463765 | A | 09 March 2021 | None | |
| CN | 101977181 | A | 16 February 2011 | None | |
| CN | 103942454 | A | 23 July 2014 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110745539X